(19) Europäisches Patentamt
European Patent Office
Office européen des brevets

(11) EP 3 684 751 B1

(12) EUROPÄISCHE PATENTSCHRIFT

(45) Veröffentlichungstag und Bekanntmachung des Hinweises auf die Patenterteilung:
**29.09.2021 Patentblatt 2021/39**

(21) Anmeldenummer: **18759330.6**

(22) Anmeldetag: **30.08.2018**

(51) Int Cl.:
***C07C 67/39*** (2006.01) ***C07C 69/54*** (2006.01)
***B01J 21/06*** (2006.01) ***B01J 23/52*** (2006.01)
***B01J 35/00*** (2006.01) ***B01J 35/02*** (2006.01)
***B01J 35/10*** (2006.01) ***B01J 37/02*** (2006.01)
***B01J 37/18*** (2006.01)

(86) Internationale Anmeldenummer:
**PCT/EP2018/073294**

(87) Internationale Veröffentlichungsnummer:
**WO 2019/057458 (28.03.2019 Gazette 2019/13)**

(54) **KATALYSATOR FÜR DIE OXIDATIVE VERESTERUNG VON ALDEHYDEN ZU CARBONSÄUREESTERN**

CATALYST FOR OXIDATIVE ESTERIFICATION OF ALDEHYDES TO CARBOXYLIC ACID ESTERS

CATALYSEUR POUR L'ESTERIFICATION OXYDATIVE D'ALDEHYDES EN ESTERS D'ACIDE CARBONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.09.2017 EP 17191731**

(43) Veröffentlichungstag der Anmeldung:
**29.07.2020 Patentblatt 2020/31**

(73) Patentinhaber: **Röhm GmbH**
**64295 Darmstadt (DE)**

(72) Erfinder:
- **KRILL, Steffen**
  **64367 Mühltal (DE)**
- **TRESKOW, Marcel**
  **Mobile, AL 36695 (US)**

(74) Vertreter: **Röhm Patent Association**
**IP Management**
**Deutsche-Telekom-Allee 9**
**64295 Darmstadt (DE)**

(56) Entgegenhaltungen:
**EP-A1- 2 210 664 EP-A1- 3 170 558**

- **LI, Y.; ZHENG, Y.; WANG, L.; FU, Z.: "Oxidative Esterification of Methacrolein to Methyl Methacrylate over Supported Gold Catalysts Prepared by Colloid Deposition", CHEMCATCHEM, Bd. 9, Nr. 11, 21. Februar 2017 (2017-02-21), Seiten 1960-1968, XP002779575,**
- **KENJO, S.; YOSHITAKE, H.: "Alkoxylation of alpha, beta-unsaturated aldehydes on gold supported on mesoporous anatase", MICROPOROUS AND MESOPOROUS MATERIALS, Bd. 237, 10. September 2016 (2016-09-10), Seiten 12-22, XP002779576,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 684 751 B1

## Beschreibung

### Gebiet der Erfindung

Die vorliegende Erfindung betrifft ein neuartiges Verfahren zur oxidativen Veresterung, allgemein zur Umsetzung von Aldehyden mit Alkoholen in Gegenwart sauerstoffhaltiger Gase direkt zum korrespondierenden Ester in Gegenwart eines heterogenen Kontakts, mittels derer beispielsweise (Meth)acrolein zu Methyl(meth)acrylat umgesetzt werden kann. Die erfindungsgemäß dazu eingesetzten Katalysatoren zeichnen sich dabei insbesondere durch eine hohe mechanische und chemische Stabilität sowie durch eine gute katalytische Performance auch über sehr lange Zeiträume aus. Dies betrifft insbesondere eine Verbesserung der Katalysatorstandzeit, der Aktivität und der Selektivität gegenüber Katalysatoren des Standes der Technik, die in Medien mit auch geringem Wassergehalt im kontinuierlichen Betrieb relativ schnell Aktivität und/oder Selektivität verlieren. Das Besondere an diesem neuen, erfindungsgemäßen Katalysator ist, dass dieser als Hauptkomponente des Trägermaterials Titandioxid aufweist. Ein weiterer wichtiger Aspekt ist die Unterdrückung und signifikante Reduktion der Bildung von Nebenprodukten, die nahe dem gewünschten Ester, insbesondere Methylmethacrylat sieden, bzw. schwer trennbare Azeotrope mit dem Produkt oder den Edukten ausbilden. Durch den neuen Katalysatortyp lassen sich so MMA Reinheiten und Qualitäten erzeugen, die deutlich höher sind, als mit den aus dem Stand der Technik bislang beschriebenen Kontakten.

### Stand der Technik

Die katalytische oxidative Veresterung von Aldehyden zur Herstellung von Carbonsäureestern ist im Stand der Technik umfänglich beschrieben (CHEMCATCHEM, Bd. 9, Nr. 11, 2017, Seiten 1960-1968 and Microporous and mesoporous materials, Bd. 237, 1016, Seiten 12-22). So kann man z.B. derart sehr effizient Methylmethacrylat aus Methacrolein (MAL) und Methanol herstellen. Insbesondere in US 5,969,178 und in US 7,012,039 wird ein Verfahren zur kontinuierlichen Herstellung von MMA aus Isobuten oder tert-Butanol beschrieben. Dabei weist das Verfahren die folgenden Schritte auf: 1) Oxidation von Isobuten oder tert-Butanol zu Methacrolein und 2) Direkte oxidative Veresterung von MAL mit Methanol zu MMA mit einem Pd-Pb-Katalysator, der sich auf einem oxidischen Träger befinden. Obwohl Umsatz und Selektivität prinzipiell hoch sind, muss für den kontinuierlichen Betrieb ständig eine Blei-Komponente zugeführt werden, da der Katalysator einen kontinuierlichen geringen Verlust von Blei-Ionen aufweist. Die Aufarbeitung und die Entfernung bleihaltiger Abwässer erfordern großen technischen Aufwand und erzeugen letztlich auch kritische schwermetallhaltige Reststoffe und Abwässer.

Jedoch weisen sämtliche aus dem Stand der Technik bekannten Katalysatoren bei längeren Standzeiten einen relevanten Verlust an Selektivität und/oder Aktivität auf. So werden zum Beispiel in EP 1 393 800 zwar gute Aktivitäten und Selektivitäten beschreiben, es wird jedoch gleichzeitig keine Auskunft über die Lebensdauer der Katalysatoren gegeben. Es handelt sich dabei um einige Gold-haltige Katalysatoren, wobei die als aktive Oxidationsspezies beschriebenen katalytischen Goldpartikel insbesondere einem durchschnittlichen Durchmesser von weniger als 6 nm aufweisen. Besagte Goldpartikel befinden sich verteilt auf einem Siliziumoxid- bzw. einem $TiO_2/SiO_2$-Träger. Als zusätzliche Aktivkomponenten außer Gold, enthalten solche Katalysatoren unter anderem auch andere Metalle. Diesen Dotierungskomponenten wird ein synergistischer und Aktivitäts- und Selektivitäts-steigernder Effekt zugeschrieben, machen aber die Katalysatorherstellung auch komplex und schwer reproduzierbar.

Die Herstellung erfolgt durch Auftragen des Goldsalzes und weiteren Metallsalzen auf einen oxidischen Träger und eine anschließende thermische Behandlung in Gegenwart von Wasserstoff als Reduktionsmittel. Für die Umsetzung von Pyruvaldehyd zu Ethylpyruvat wird dabei z.B. auch ein Gold- und Kobalt-haltiger Katalysator auf einem $TiO_2$-Träger beschrieben. Dabei liegt Kobalt in diesem Katalysator in metallischer Form {Co(0)} vor. Die Selektivität zum Zielprodukt (Ethylpyruvat) beträgt in diesem Fall 81% bei Raum-Zeit-Ausbeute von 24 mol/kg Kat*h. Die Selektivitäten von anderen Gold-haltigen Katalysatoren (ohne Kobalt) zu MMA werden bei einem Gehalt von 4,5 Gew% Au mit bis zu 93% und die Raum-Zeit-Ausbeute mit bis zu 50,7 mol MMA/kg Kat*h angegeben. Naturgemäß ist die Verwendung solch hoher Goldbeladungen zur Erzielung hoher Raum Zeit Ausbeuten mit erheblichen Kosten bei der Katalysatorherstellung verbunden.

Haruta et al beschreiben in J. Catal. 1993, Vol. 144, pp 175-192, dass Goldnanopartikel aufgetragen auf übergangsmetalloxidischen Trägern, wie $TiO_2$, $Fe_2O_3$ oder $Co_3O_4$ aktive Oxidationskatalysatoren darstellen. Dabei spielt eine Wechselwirkung zwischen Gold und Übergangsmetall eine entscheidende Rolle für die Katalysatoraktivität.

In der US 6,040,472 werden alternative Katalysatoren, die jedoch im Vergleich nur zu unzureichenden Aktivitäten und Selektivitäten zu MMA führen, beschrieben. Es handelt sich in diesem Fall um Pd-Pb-haltige Katalysatoren mit Schalenstruktur. Die Selektivitäten zu MMA werden mit bis zu 91 % und die Raum-Zeit-Ausbeute mit bis zu 5,3 mol angegeben. Auch hier ist die Blei Dotierung für die Ausbildung der aktiven Oxidationsspezies entscheidend, erzeugt aber durch schleichenden Blei Ionen Verlust die oben beschriebenen Nachteile, die Katalysatorherstellung ist vergleichsweise kompliziert, auch und insbesondere durch die Verwendung kritischer Bleisalze bei der Imprägnierung.

In EP 2 177 267 und EP 2 210 664 werden nickelhaltige Katalysatoren mit Schalenstruktur beschrieben. Selektivität zu MMA beträgt bei diesen Katalysatoren bis zu 97%. Die Raum-Zeit-Ausbeute wird mit 9,7 mol MMA/(kg h) bei einem Goldanteil im Katalysator von ca. 1 Gew% beschrieben. Ein $NiO_x$/Au-Katalysator zeigt laut Beispielen deutlich bessere Aktivitäten und Selektivitäten zu MMA, während andere Kombinationen, wie z.B. Au mit CuO oder auch $Co_3O_4$ viel weniger aktiv und selektiv sind.

Auch beschreibt die EP 2 177 267 in Vergleichsbeispiel 7 eine Herstellung von Au/$Co_3O_4$ haltigen Katalysatoren, ausgehend von Kobaltnitrat und Goldsäure durch gleichzeitiges Auftragen von Au und Co auf einen $SiO_2$/MgO-Träger. Diese Methode der Auftragung führt erfahrungsgemäß für NiO/Au-Katalysator zu den besten Ergebnissen. Nicht jedoch bei der Verwendung von Kobalt, da hier mit der Verwendung des resultierenden Katalysators zur Herstellung von MMA aus Methacrolein nur 2,6% Umsatz und 45,8% Selektivität bei einer Raum-Zeit-Ausbeute (RZA) von 0,3 mol/(kg h) erzielt werden. Das Vergleichsbeispiel 6 des gleichen Patents beschreibt die Synthese und Verwendung eines Au/$Fe_3O_4$-Katalysators für die gleiche Umsetzung. Mit diesem Katalysator werden dann 10,4% Umsatz und 55,2% Selektivität an MMA bei einer RZA von 1,4 mol/(kg h) erreicht. Für den industriellen Einsatz sind diese Katalysatoren daher eher nicht geeignet.

Die EP 2 210 664 offenbart einen Katalysator, der im Außenbereich, in Form einer so genannten Egg-Shell-Struktur, Nickeloxid und Goldnanopartikel auf einem Träger aus $SiO_2$, $Al_2O_3$ und einem basischen Element, insbesondere einem Alkali- oder Erdalkalimetall, aufweisen. Das Nickeloxid ist dabei an der Oberfläche angereichert, jedoch auch in tieferen Schichten des Katalysatorpartikels in geringeren Konzentrationen enthalten. Ein solcher Katalysator zeigt sehr gute Aktivitäten und Selektivitäten. Jedoch ist der Katalysator hergestellt nach der erfindungsgemäßen Herstellungsvorschrift aus dieser Anmeldung relativ empfindlich für Abrieb und instabil, was ein Vergleichsbeispiel im weiteren Text zeigt. Dadurch hat man nur relativ geringe Standzeiten zur Verfügung. Die besondere Herstellmethode zu Erzeugung der Egg-Shell Struktur, die Verwendung von nicht unkritischen Ni-Salzen bei der Herstellung des Katalysators stellen besondere Anforderungen an technische Apparate und das Handling von Ni-haltigen Feinstäuben wie sie zwangsläufig bei der Katalysatorfertigung anfallen, z.B. beim Prozessschritt Trocknung und Kalzinierung. Auch hier wird die Dotierungskomponente Nickel neben Gold Nanopartikeln und die besondere anisotrope, inhomogene Verteilung von Gold und Dotierung als notwendig beschrieben, um hohe Aktivität und Selektivität zu erreichen.

Xiaoyue Wan, Catalysis today, 233, 2014, S. 147 (Tab. 1) lehrt, Gold-basierte Katalysatoren, die auf Trägern, die aus Titandioxid bestehen, aufgebracht sind, würden bei der oxidativen Veresterung in einem Batch-Prozess sehr schlechte oder gar keine Ausbeuten bei äußerst geringer Selektivität erbringen.

C. Marsden, C. H. Christensen et al. beschreiben in Green Chem., 2008, 10, 168-170 die direkte oxidative Veresterung (kurz DOE) verschiedener Aldehyde in Gegenwart verschiedener Katalysatoren, die nanopartikuläres Gold auf diversen Trägern aufweisen. Acrolein wird hier zu Methylacrylat an einem Au/ZnO Katalysator umgesetzt, allerdings ist die Reaktionszeit mit bis zu 40 Stunden zum Erzielen von Umsätzen von 90% unwirtschaftlich für eine technische Anwendung, zumal nur eine Selektivität von 87% erreicht wird.

In Fortsetzung dieser Arbeiten hat Mc Pherson et al. für die Fa. Mintec mit verschiedenen nanopartikulären goldhaltigen Katalysatoren ebenfalls die Acrolein DOE zu Methylacrylat untersucht (http://www.mintek.co.za/Mintek75/Proceedings/J04-McPherson.pdf). Hier werden auf TiO2 geträgerte Au Katalysatoren in Batchversuchen eingesetzt, wobei ebenfalls bei hohen Umsätzen nur Selektivitäten von 87% bei extrem langen Reaktionszeiten erreicht werden können. Bei niedrigeren Umsätzen ist die Selektivität deutlich schlechter mit 67% angegeben und somit wirtschaftlich uninteressant. Auf Basis dieser Ergebnisse erschien eine Übertragung auf ein kontinuierliches Verfahren nicht wirtschaftlich.

EP 1 393 800 lehrt wiederum, dass ein Gold-dotierter Katalysator auf einem Träger aus Silika und Titandioxid zwar eine akzeptable Ausbeute herbeiführen kann, dabei aber sehr unselektiv ist und große Mengen Nebenprodukte erzeugt. Der entsprechende Katalysator hatte einen Titan-Anteil von unter 5 Gew%. Entsprechende relativ unbefriedigende Ergebnisse für $TiO_2$-dotierte Träger eines Gold- bzw. Gold/Nickeloxid-basierten Katalysators finden sich in Ken Suzuki, ACS Catalysis., 3(8), 2013, S.1845.

Alle im Stand der Technik beschriebenen Systeme, Prozesse und Katalysatoren beschreiben zudem nicht oder nur ungenügend die Bildung kritischer, insbesondere u.a. hydrierter Nebenprodukte, die bei der Isolierung von marktüblichen Qualitäten von Alkylmethacrylaten, insbesondere MMA eine wesentliche Rolle spielen. Ein solches kritisches Nebenprodukt, das nur mit erheblichem apparativen Aufwand und erheblichem Energieeinsatz von MMA getrennt werden kann, ist Methylisobutyrat, auch als Isobuttersäuremethylester bezeichnet.

Diese Nebenprodukte sind die korrespondierenden gesättigten Kohlenwasserstoffe von im Verfahren hergestellter ungesättigter Verbindungen, wie beispielsweise der hydrierten Folgeprodukte der Alkylmethacrylate, und haben meist sehr ähnliche Siedepunkte oder Azeotrope, was eine destillative Abtrennung sehr aufwendig macht und mitunter nur unter Verlust von Ausbeute möglich ist.

Diese Komponente kommt bei vielen gängigen technischen Methylmethacrylatverfahren vor und befindet sich letztlich auch im Verkaufsprodukt MMA, wie es beispielsweise für die Herstellung von PMMA eingesetzt wird, allerdings in Konzentrationen deutlich kleiner 500 ppm, üblicherweise auch kleiner als 100 ppm. Insgesamt besteht also weiterhin

Bedarf bessere Katalysatoren für die oxidative Veresterung von Aldehyden mit Alkoholen zur Verfügung zu stellen, insbesondere der Herstellung von MMA durch direkte oxidative Veresterung von Methacrolein mit Methanol in Gegenwart sauerstoffhaltiger Gasmischungen, die einen möglichst geringe Erzeugung des schwer abtrennbaren Methylisobutyrats aufweisen. Somit besteht insgesamt ein großes Interesse an Katalysatoren, die zu hohen Ausbeuten bei gleichzeitig hohen Selektivitäten und langen Reaktorstandzeiten führen bei gleichzeitig minimierter Erzeugung von Nebenkomponenten wie Methylisobutyrat, die nur aufwändig vom Monomer abtrennbar sind.

Gängige Katalysatoren zur Erfüllung der Aufgabe, auf Basis von Nickel-Gold Katalysatoren (EP 2177267 und EP2210664) und Cobalt-Gold Katalysatoren (WO 2017084969) erzeugen ein Reinprodukt nach Aufarbeitung mit einem Gehalt an Isobuttersäuremethylester im Bereich von 500-1000ppm. Es ist aus der Literatur hinreichen bekannt, dass sowohl Nickel als auch Cobalt hervorragende Hydrierungskatalysatoren sind. (Vgl Raney Nickel und Raney Cobalt aus: Lehrbuch der Anorganischen Chemie, Arnold Fr. Holleman, Egon Wiberg, Gruyter, ISBN: 9783110126419).

Insgesamt werden also diverse Katalysatoren im Stand der Technik beschrieben für die Direkte Oxidative Veresterung (DOE) zum Beispiel für die Umsetzung von ungesättigten Aldehyden wie Acrolein und Methacrolein. Die Rohstoffe, insbesondere die ungesättigten Aldehyd-Verbindungen, werden in industriell ausgeübten Verfahren hergestellt. Acrolein wird üblicherweise durch eine Gasphasen-Partialoxidation an heterogenen Kontakten aus Propylen bzw. Propan erhalten.

Methacrolein kann ebenfalls auf verschiedenen Wegen industriell erzeugt werden. Beispielsweise wird Methacrolein durch eine Gasphasen-Partialoxidation an einem heterogenen Kontakt aus einem C-4 Rohstoff hergestellt, im einfachsten Fall aus Isobuten, das wiederum aus MTBE durch Eliminierung von Methanol aus MTBE (Methyl-tert-butylether) zugänglich ist. In wiederum einer weiteren Verfahrensvariante wird tert-Butylalkohol als C-4 Rohstoff verwendet, das direkt auf die heterogenen Oxidationskatalysatoren gasförmig gegeben wird, optional an einem Vor-Kontakt gasförmig dehydratisiert wird. Isobutan kommt ebenfalls als Rohstoff für die intermediäre Herstellung von Methacrolein in Frage und ist beschrieben. Einen guten Überblick über die Herstellung von Methacrolein nach diesen C-4 Verfahrens Varianten gibt die Publikation von Nagai und Ui, "Trends and Future of Monomer-MMA Technologies", Sumitomo Basic Chem. Research Lab. (http://www.sumitomo-chem.co.jp/english/rd/report/theses/docs/20040200_30a.pdf)

Methacrolein kann auch aus Ethylen als C-2 basierten Rohstoffen anstelle von C-4 basierten Ausgangsmaterialien gewonnen werden. Dazu wird Ethylen zunächst mit Synthesegas zu Propanal umgesetzt. Aus diesem erhält man in einer zweiten Stufe zusammen mit Formaldehyd dann Methacrolein. Dieser Ansatz kann beispielsweise in Ullmann's Encyclopedia of Industrial Chemistry 2012, Methacrylic Acid from Ethylene, and Trends and Future of Monomer-MMA Technologies, SUMITOMO KAGAKU 2004-II nachgelesen werden. Besondere Ausführungsformen der Reaktion können beispielsweise in EP 0 092 097 oder DE 28 55 504 nachgelesen werden. Das resultierende Methacrolein wird anschießend zu Methacrylsäure oxidiert und abschließend mit einem Alkylalkohol zu einem Alkylmethacrylat verestert.

Insgesamt besteht also weiterhin Bedarf bessere Katalysatoren für die oxidative Veresterung von Aldehyden mit Alkoholen zur Verfügung zu stellen. Dabei besteht weiterhin ein großes Interesse an Katalysatoren, die zu hohen Ausbeuten bei gleichzeitig hohen Selektivitäten und langen Reaktorstandzeiten führen. Die allgemeine technische Lehre dabei ist, dass insbesondere Titandioxid-geträgerte als auch mit Titandioxid dotierte Trägerkatalysatoren dazu insbesondere ungeeignet sind.

## Aufgabe

Aufgabe der vorliegenden Erfindung war primär, ein neues Verfahren zur Synthese von Alkylmethacrylaten, basierend auf einem neuartigen Katalysator, für eine hoch selektive oxidative Veresterung von Aldehyden zu Carbonsäureestern zur Verfügung zu stellen. Dabei soll dieser Katalysator eine hohe mechanische und chemische Stabilität, insbesondere in Wasser- und Carbonsäure-haltigen Gemischen, aufweisen, und über ein gegenüber dem Stand der Technik insgesamt besseres Gesamtbild aus Aktivität, Selektivität und Lebensdauer unter Produktionsbedingungen verfügen.

Insbesondere bestand die Aufgabe, dass dieses Verfahren für die oxidative Veresterung von Methacrolein zu einem Alkylmethacrylat, insbesondere zu MMA geeignet sein soll.

Ein besonders wichtiger Teilaspekt der Aufgabenstellung, die der vorliegenden Erfindung zugrunde lag, war es, ein neuartiges Verfahren zur Verfügung zu stellen, welches insbesondere bei der Umsetzung von Aldehyden zu Carbonsäureestern zu einer verminderten Bildung von Nebenprodukten und damit zu einer höheren Selektivität führt. Solche Nebenprodukte sind zum Beispiel Methacrylsäure oder auch Alkoxy-iso-buttersäurealkylester, im Falle der MMA-Synthese Methoxy-iso-buttersäuremethylester (MIBSM).

Ein besonders wichtiger Teilaspekt der Aufgabenstellung, die der vorliegenden Erfindung zugrunde lag, war es, ein neuartiges Verfahren zur Verfügung zu stellen, welches insbesondere bei der Umsetzung von Aldehyden zu Carbonsäureestern zu einer verminderten Bildung von Nebenprodukten, insbesondere hydrierten Nebenprodukten führt, und damit zu einer höheren Produktreinheit führt. Gesucht wird somit insbesondere nach einem Katalysator der eine Hydrierung der Endprodukte nicht oder nur stark eingeschränkt ausführt.

Weitere nicht explizit genannte Aufgaben können sich aus der Beschreibung, den Beispielen, den Ansprüchen oder dem Gesamtzusammenhang der vorliegenden Erfindung ergeben.

**Lösung**

Gelöst wurden die gestellten Aufgaben mit Hilfe eines neuartigen Verfahrens für die oxidative Veresterung von Aldehyden zu Carbonsäureestern, in dem für das Verfahren gänzlich neue, in Hinblick auf die Lehre des Standes der Technik sogar überraschend effiziente und langlebige Katalysatorpartikel, eingesetzt werden. Bevorzugt handelt es sich bei dem Verfahren um eine oxidative Veresterung eines Aldehyds in Gegenwart von Sauerstoff und einem Alkohol zu einem Carbonsäurester. Bei dem erfindungsgemäßen Verfahren kann es sich besonders bevorzugt um ein Verfahren für die oxidative Veresterung von Methacrolein in Gegenwart von sauerstoffhaltigen Gases und einem Alkohol zu einem Alkylmethacrylat. insbesondere von Methacrolein in Anwesenheit von Methanol zu MMA handeln.

Alternativ kann bei dieser Verwendung auch Methacrolein mit Sauerstoff und einem di-, tri- oder tetra-funktionellen Alkohol zu einem Hydroxyalkylmethacrylat und di-, tri- bzw. tetra-Methacrylat umgesetzt werden. Die letzteren Verbindungen sind als Vernetzer bekannt. Ein besonders bevorzugtes Beispiel für einen di-funktionellen Alkohol ist Ethylenglycol.

Diese erfindungsgemäß in dem Verfahren verwendeten Katalysatoren sind dabei dadurch gekennzeichnet, dass das Katalysatorpartikel aus 0,1 bis 3 Gew%, bevorzugt 0,3 bis 2,5 Gew% Gold, 25 bis 99,8 Gew%, bevorzugt 50 bis 99,5 Gew% $TiO_2$, 0 bis 50 Gew%, bevorzugt 0 bis 20 Gew% Siliziumoxiden, 0 bis 25 Gew% $Al_2O_3$, 0 bis 25 Gew% Oxiden von Alkalimetallen, Erdalkalimetallen Seltenerdemetallen und/oder Zirkonium, 0 bis 20 Gew%, bevorzugt 0 bis 10 Gew% Eisen-, Zink-, Nickel- und/oder Kobaltoxiden und 0 bis 5 Gew% weiteren Komponenten besteht. In der Regel liegt das Gold dabei elementar in Form von Au{0} und in Form von Nanopartikeln vor, während alle anderen Komponenten im Katalysatorpartikel in oxidischer Form vorliegen.

Darüber hinaus zeichnen sich diese erfindungsgemäß in dem Verfahren verwendeten Katalysatorpartikel dadurch aus, dass das Gold, optional zusammen mit Eisen-, Zink-, Nickel- und/oder Kobaltoxiden in Form von Partikeln - auch als Primärpartikel bezeichnet - mit einem mittleren Durchmesser zwischen 2 und 10 nm vorliegt und dabei diese Partikel an die Oberfläche des übrigen Katalysatorpartikels gebunden sind.

Dies bedeutet im Falle von Mischphasen des Goldes mit den genannten Oxiden dabei jedoch nicht zwangsläufig, dass diese Oxide gänzlich Teil der Partikel mit einem mittleren Durchmesser zwischen 2 und 10 nm im Katalysatorpartikel vorliegen müssen. Vielmehr kann auch ein Teil der Oxide Teil des Trägermaterials sein und beispielsweise anderweitig in irgendeiner Form vor allem an die Oberfläche des Trägermaterials gebunden sein. Besonders bevorzugt weist der Katalysatorpartikel jedoch kein Eisen-, Zink-, Nickel- und/oder Kobaltoxid auf, sondern ausschließlich Gold als einzige aktive Komponente. Das erfindungsgemäß in dem Verfahren eingesetzte Katalysatorpartikel hat einen mittleren geometrischen Äquivalenzdurchmesser zwischen 5, bevorzugt 10 $\mu$m und 10 mm, besonders bevorzugt zwischen 200 $\mu$m und 5 mm.

Je nach Herstellungsverfahren ist es sowohl möglich, dass das Gold in Form reiner Partikel als auch in einer Mischform z.B. mit einem genannten Oxid, wie beispielsweise Kobaltoxid vorliegt. Dabei ist das Gold in letzterem Fall in der Regel nur mit einem Teil des Oxids gemischt. Weiterhin ist es in beiden Ausführungsformen optional auch möglich, dass die Gold- bzw. goldhaltigen Partikel zur Stabilisierung zusätzlich mit einer dünnen Schicht, z.B. aus $TiO_2$, $SiO_2$ und/oder $Al_2O_3$ versehen werden.

Darüber hinaus wird das erfindungsgemäße Verfahren kontinuierlich, mittels einer heterogenen Katalyse in einer Flüssigphase durchgeführt. Dabei liegt bevorzugt in dem kontinuierlich betriebenen Reaktor ein dreiphasiges System mit einer festen Katalysatorphase, einer flüssigen Reaktionsphase und einer Gasphase vor.

In einer besonderen Ausführungsform der vorliegenden Erfindung, ist das Verfahren dadurch gekennzeichnet, dass der Katalysatorpartikel überwiegend oder sogar ausschließlich aus Gold und $TiO_2$ zusammengesetzt ist. In dieser Variante besteht der Katalysatorpartikel bevorzugt aus 0,3 bis 2,5 Gew% Gold, 96 bis 99,5 Gew% $TiO_2$ und maximal 3,7 Gew%, besonders bevorzugt 1,5 Gew% weiterer Komponenten, bei denen es sich weder um Gold noch um $TiO_2$ handelt.

Bevorzugt sind die erfindungsgemäß verwendeten Katalysatorpartikel porös. Wobei sich die Porosität in der Regel nicht auf die Gold- bzw. goldhaltigen Phasen bezieht. Dabei weisen solche porösen Katalysatorpartikel eine spezifische Oberfläche zwischen 20 und 300 $m^2/g$, bevorzugt zwischen 30 und 200 $m^2/g$, besonders bevorzugt zwischen 80 und 150 $m^2/g$ auf. Weiterhin beträgt in der Regel der durchschnittliche Porendurchmesser dabei 2 bis 50 nm, bevorzugt 5 bis 30 nm, besonders bevorzugt 10 bis 25 nm.

Eine optionale Komponente des erfindungsgemäß eingesetzten Katalysatorpartikels sind so genannte basische Elemente. Bei diesen basischen Elementen handelt es sich insbesondere um Alkalimetalle (Li, Na, K, Rb, Cs, Fr), Erdalkalimetalle (Be, Mg, Ca, Sr, Ba), Seltenerdemetalle (Sc, Y, La, Ce. Pr, Nd, Pm, Sm, Eu, Gd, Tb, Dy, Ho, Er, Tm, Yb, Lu) oder um Mischungen aus diesen Metallen. Das basische Element liegt dabei in der Regel auch als Oxid vor. Es ist bei einem längeren Betrieb des Verfahrens mit dem erfindungsgemäß verwendeten Katalysatorpartikeln durchaus

möglich, dass ein frisch hergestellter Katalysator während eines Prozesses zur Herstellung von Carbonsäureestern einen Teil des basischen Elements verlieren kann. Dies kann je nach Art des Katalysators sowohl zu einer geringfügigen Verbesserung als auch zu einer Verschlechterung der Aktivität und/oder Selektivität des Prozesses führen und begründet sich durch während des Prozesses als Nebenprodukt gebildeten Säuren.

Weitere geeignete Metalloxide, insbesondere für den Trägeranteil des Katalysators stellen Zirkoniumoxide dar.

Bei den optional mit bis zu 5 Gew% in dem Katalysatorpartikel vorhandenen weiteren Komponenten, handelt es sich um Komponenten, die zunächst einmal von den sonst aufgeführten Verbindungen bzw. Elementen abweichen. Ein insbesondere geeignetes Beispiel für eine solch weitere Komponente ist Zirkonium, bzw. eine Zirkoniumverbindung, wie insbesondere Zirkoniumoxid.

In einer bevorzugten Ausführungsform der vorliegenden Erfindung liegen die goldhaltigen Primärpartikel im Außenbereich des Katalysatorpartikels vor. In einer zweiten, gleichfalls bevorzugten Ausführungsform sind diese Primärpartikel gleichmäßig über den Katalysatorpartikel verteilt.

In der ersten genannten Ausführungsform sind die erfindungsgemäß in dem Verfahren verwendeten Katalysatorpartikel dadurch charakterisiert, dass die maximale Gold- bzw. die maximale Eisen-, Zink- oder Kobaltkonzentration des Katalysatorpartikels im Außenbereich desselben zu finden ist. Der besagte Außenbereich macht dabei maximal 60 %, bevorzugt maximal 40 % und besonders bevorzugt maximal 30 % des geometrischen Äquivalenzdurchmessers des Katalysatorpartikels aus. Dabei ist die Gold- bzw. Eisen-, Zink- und/oder Kobaltkonzentration in diesem Außenbereich mindestens 1,5 mal, bevorzugt mindestens zweimal und insbesondere bevorzugt mindestens 2,5 mal höher ist als die entsprechende Konzentration dieser Elemente im mittleren Bereich, der den verbliebenen Bereich des geometrischen Äquivalenzdurchmessers des Katalysatorpartikels ausmacht. Besonders bevorzugt befindet sich das Gold in dieser Ausführungsform zu mehr als 90% in diesem Außenbereich. Im Falle eines solchen Aufbaus des Katalysatorpartikels spricht man von einer so genannten Egg-Shell-Struktur.

Die Ermittlung und Analyse der Verteilung der Konzentrationen von Gold und/oder Eisen-, Nickel-, Zink- und/oder Kobalt entlang des Katalysatorpartikelprofils kann z.B. durch die Einbettung der Partikeln in eine Polymermatrix, nachfolgende Polierung und anschließende REM-EDX Analyse erfolgen. Eine analoge Analysemethode mittels Röntgen Microprobe (EPMA) wird z.B. in EP 2 210 664 A1 auf Seite 18 beschrieben.

Die erfindungsgemäß verwendeten Katalysatorpartikel und damit das erfindungsgemäße Verfahren zeichnen sich insbesondere in Hinblick auf den zuvor diskutierten Stand der Technik durch eine Reihe von zuvor nicht zu erwartenden Vorteilen aus. So zeichnen sich diese Katalysatorpartikel insbesondere durch den Erhalt einer hohen Aktivität bei einer gleichzeitig hohen Selektivität des für die oxidative Veresterung eingesetzten Katalysatorpartikels über eine lange Zeit aus. Damit weisen die erfindungsgemäß verwendeten Katalysatorpartikel eine besonders gute Kombination aus a) einem geringen mechanischen Abrieb des Katalysatorpartikels, b) einem geringen Leaching von Metallionen, die beispielsweise im Falle von Eisen in einem erfindungsgemäß hergestellten MMA zu Problemen bezüglich der Stabilität führen können, aus dem Partikel und c) einem langfristigen Erhalt der Katalysatorleistung bzgl. Aktivität und Selektivität.

Besonders überraschend wurde festgestellt, dass die erfindungsgemäßen Katalysatoren nicht nur zu einer generell höheren Selektivität führen, sondern dass insbesondere die Bildung besonders unerwünschter Nebenprodukte, wie zum Beispiel von Methacrylsäure, unterdrückt wird. Genauso wurde zum Beispiel im Falle der oxidativen Veresterung von Methacrolein mit Methanol zu MMA eine weiterhin verminderte Bildung von Methoxy-iso-Buttersäuremethylester (MIBSM) festgestellt.

Darüber hinaus wurde überraschend festgestellt, dass mit den erfindungsgemäß eingesetzten Katalysatoren die Bildung hydrierter Folgeprodukte der angestrebten Hauptprodukte gegenüber dem Stand der Technik deutlich vermindert werden konnte. So wird erfindungsgemäß die Bildung von z.B. hydriertem MMA bei der Verwendung eines erfindungsgemäßen $TiO_2$-basierten Katalysators gegenüber den im Stand der Technik beschriebenen besten Katalysatoren um den Faktor 10 von einem Gehalt zwischen 500 und 1000 ppm auf einen Gehalt zwischen 50 und 100 ppm reduziert.

Viele der Nebenprodukte der Reaktion bilden sich insbesondere bei hohen Konzentrationen der Ausgangsmaterialien. Dimeres Methacrolein und dessen Ester z.B. bilden sich bevorzugt bei hohen Konzentrationen des Ausgangsmaterial Methacrolein. Dimethoxyisobuten bildet sich insbesondere bei neutralen und sauren Bedingungen bei hohen Konzentrationen von Methanol und Methacrolein. 3-Methoxyisobuttersäuremethylester (kurz 3-MibsM) bildet sich bei hohen Konzentrationen des Methanols als Michaelprodukt mit Methacrolein (und nachfolgender direkter oxidativen Veresterung), besonders bei neutral und basischen Bedingungen.

Der Bildung dimerer Methacroleine (kurz MAL-Dimer) kann mit höheren pH-Werten derart entgegengesteuert werden, dass bei hohen pH-Werten der Umsatz des MAL höher und die Konzentration desgleichen damit geringer ist. Dies resultiert in einer verminderten Bildung des MAL-Dimers bzw. deren Ester. Die Änderung des pH-Werts würde jedoch auf der anderen Seite nach Stand der Technik die bevorzugte Bildung des anderen Nebenprodukts 3-MibsM steigern. Erfindungsgemäß spielt die Bildung des MAL-Dimers jedoch eine überraschend untergeordnete Rolle, so dass es jetzt möglich wird, die Ausbeute und Selektivität des Verfahrens überraschend mit steigendem Umsatz zu verbessern. Dies erfolgt derart, dass ein zusätzlicher überraschender Effekt erfindungsgemäß darin besteht, dass das Verfahren

bei besonders hohem Umsatz zu betreiben ist, ohne dass dabei die Verweilzeit im Reaktor zu verlängern wäre.

Die Diels-Alder-Reaktion, die zur Bildung des dimeren MAL bzw. dessen Ester führt, wird insbesondere auch durch eine höhere Temperatur begünstigt. Erfindungsgemäß kann dem überraschend durch gemäßigtere Reaktionsbedingungen zusätzlich entgegengesteuert werden. Genauso überraschend wird auch die Bildung eines Isobuttersäuremethylester aus Hydrierungs-Nebenreaktion deutlich vermindert, so dass der vorliegende Katalysator in einem kontinuierlichen Verfahren nach einer Einlaufzeit eine überraschend hohe Selektivität auf konstantem Niveau über eine sehr lange Betriebsdauer behält.

Bevorzugt weisen die Katalysatorpartikel einen mittleren geometrischen Äquivalenzdurchmesser zwischen 10 μm, bevorzugt 100 μm und 5 mm, besonders bevorzugt 500 μm und 2 mm auf. Dabei bezieht sich der Durchmesser auf den mittleren geometrischen Äquivalenzdurchmesser (im Weiteren auch als mittlerer Äquivalenzdurchmesser oder geometrischer Äquivalenzdurchmesser gleichbedeutend bezeichnet) in Form eines Volumenäquivalenten Kugeldurchmessers. Der mittlere Äquivalenzdurchmesser eines solchen ungleichmäßigen Körpers kann dabei derart bestimmt werden, dass der mittlere Äquivalenzdurchmesser dem Durchmesser $d_v$ einer dem ungleichmäßigen Körper volumengleichen Kugel entspricht. Dazu wird zunächst - unter Vernachlässigung der Porösität - das Volumen des Katalysatorpartikels bestimmt. Dies kann bei einfachen, gleichmäßigen Körpern, wie beispielsweise regelmäßig geformten Zylindern - durch einfaches Ausmessen z.B. einer mikroskopischen Aufnahme erfolgen. Bei ideal-kugelförmigen Partikeln kann die Bestimmung sogar ganz entfallen, da das Ergebnis dem tatsächlichen Kugeldurchmesser entspricht.

Im Falle sehr ungleichmäßig geformter Partikel bestimmt man das Volumen und - sollte dies porösitätsabhängig erfolgt sein - korrigiert das Ergebnis um die zusätzlich zu bestimmende Porösität.

Die Formel zur Berechnung des mittleren Äquivalenzdurchmessers, bei dem es sich erfindungsgemäß im Genaueren um den volumenäquivalenten Kugeldurchmesser $d_v$ handelt, lautet in Anhängigkeit vom Volumen V:

$$d_v = \sqrt[3]{\frac{6 * V}{\pi}}$$

Im Falle, dass sich die goldhaltigen Partikel nahe der Oberfläche des Katalysatorpartikels befinden, wird der aktive äußere Bereich eines solchen Katalysatorpartikels mit Egg-Shell-Struktur im weiteren als Außenbereich bezeichnet. Die Dicke dieses Außenbereichs liegt dabei bevorzugt zwischen 2 und 100 μm, bevorzugt zwischen 5 und 50 μm. Die Größe des geometrischen Äquivalenzdurchmessers wird dabei angegeben, da die Partikel nicht zwingend gänzlich sphärisch vorliegen müssen, sondern durchaus auch komplexere Formen aufweisen können.

Auch sei darauf hingewiesen, dass die so betrachtete Grenze zwischen dem Kern und einer Schale im Falle einer solchen optionalen Egg-Shell-Struktur weiterhin nicht scharf sein wird, sondern insbesondere in Form eines Gradienten mit sich ändernder Zusammensetzung vorliegen kann. So kann beispielsweise die Konzentration an Goldnanopartikeln vom Kern aus gesehen von innen nach außen zunehmen. Dies ergibt sich allein schon aus der Tatsache, dass die erfindungsgemäßen Partikel in der Regel eine Porosität aufweisen. Ein beispielhafter Wert von 80 μm Dicke des Außenbereichs, z.B. bei einem Partikel mit einem Äquivalenzdurchmesser von 200 μm bedeutet im Idealfall, dass sich über den Durchmesser gesehen an den beiden Außenenden desselben jeweils 40 μm Außenbereich und dazwischen 120 μm mittleren Bereich befinden. Diese Größe wurde gewählt um die Egg-Shell-Struktur des erfindungsgemäßen Katalysators zu beschreiben. Die Grenze zwischen Außen- und Innenbereich kann dabei vom Fachmann bei der Untersuchung der Partikel, in den angegeben Bereichen relativ frei gewählt werden. Entscheidend ist erfindungsgemäß, dass sich innerhalb des angegebenen Bereichs eine Grenze findet, an der die Bedingungen bezüglich der Eisen-, Zink-Nickel- und/oder Kobalt- und der Goldkonzentration gegeben sind. Dies ist in Bezug auf die Eisen, Zink bzw. insbesondere die Kobaltkonzentration der erfinderische Kern der vorliegenden Erfindung.

Im Folgenden werden die Parameter einer besonders bevorzugten Ausführungsform der vorliegenden Erfindung beschrieben. Bei Einhaltung der hier angegebenen Einstellungen erhält man besonders gute Ergebnisse des Verfahrens in Bezug auf Ausbeute und Selektivität über eine lange Betriebsdauer. Dies soll aber nicht bedeuten, dass es nicht alternative Einstellungen der vorliegenden Erfindung, die zu ähnlichen Ergebnissen führen, gibt. Es sei vielmehr darauf hingewiesen, dass der erfindungsgemäß verwendete Katalysator in Breite überraschend gute Ergebnisse im Vergleich zum Stand der Technik erbringt.

Insbesondere zeichnet sich das Verfahren dieser besonders bevorzugten Ausführungsform dadurch aus, dass die Reaktion zwischen Methacrolein und einem Alkylalkohol, insbesondere Methanol, in Gegenwart eines sauerstoffhaltigen Gases unter Einhaltung der folgenden Parameter kontinuierlich durchgeführt wird:

I. bei einer Reaktionstemperatur zwischen 40-120°C, besonders bevorzugt zwischen 50 und 100°C,
II. bei einem absoluten Druck zwischen 1 und 20 bar, besonders bevorzugt zwischen 2 und 15 bar,
III. mit einer Slurry Dichte des Katalysators, insbesondere partikulären Katalysators, der in der stationären Produktmischung, enthaltend MMA, Methacrolein, Methanol und Wasser, gerührt und verwirbelt vorliegt, zwischen 1 und 20 Gew%, besonders bevorzugt zwischen 2 und 15 Gew%.

Dabei wird bevorzugt das Edukt Methacrolein in jeweils mindestens einem Reaktor nicht vollständig umgesetzt. Vielmehr wird bevorzugt nach einem Partialumsatz zwischen 20 und 95% dieses vom Katalysator mittels Filtration kontinuierlich abgetrennt und der Aufarbeitung zugeführt. In der Aufarbeitung kann das verbliebene Methacrolein dann beispielsweise isoliert und in die Reaktion als Edukt zurückgeführt werden.

Besonders wirtschaftlich ist das Verfahren überraschend dadurch zu gestalten, wenn der dem Reaktor kontinuierlich zugeführte molare Überschuss von Methanol zu Methacrolein auf 1:1 bis 15:1 begrenzt wird. Das Konzept der "kontinuierlichen Zuführung" umschreibt hier einen für die Reaktion angemessenen Zeitraum, unabhängig davon ob Methacrolein und Methanol gleichzeitig oder sequentiell zugegeben werden. Sowohl Methacrolein als auch Methanol werden unter Verwendung der neuen Katalysatorsysteme im ebenfalls erfindungsgemäßen Verfahren nur teilweise umgesetzt. Das heißt, dass das resultierende Reaktionsgemisch nach Abtrennung vom Katalysatorpartikel derart aufgereinigt wird, dass nicht umgesetztes Methacrolein und Methanol vom Produktestern abgetrennt werden und der Reaktion erneut zugeführt werden.

Besonders bevorzugt wird Luft als sauerstoffhaltiges Gas kontinuierlich dem Reaktor zugeführt. Gleichsam besonders bevorzugt beträgt der Sauerstoffgehalt der sich über der Reaktionsmischung bildenden und befindlichen Gasphase zwischen 2 und 8 Vol%.

In einer ganz besonderen Variante der Reaktion wird das Edukt Methacrolein in mehreren in Reihe geschalteten Reaktoren mit dem Alkylalkohol umgesetzt. Auch hierbei ist es insbesondere bevorzugt, den Umsatz nicht vollständig zu erzielen, sondern nach einem Partialumsatz zwischen 20 und 90%, insbesondere bevorzugt zwischen 40 und 80% das Methacrolein vom Katalysator mittels Filtration kontinuierlich zu trennen und der Aufarbeitung zuzuführen.

In einer genauso bevorzugten, alternativen Variante der Reaktion wird das Edukt Methacrolein in genau einem Reaktor mit dem Alkylalkohol umgesetzt. Auch hierbei ist es insbesondere bevorzugt, den Umsatz nicht vollständig zu erzielen, sondern nach einem Partialumsatz zwischen 20 und 90%, insbesondere bevorzugt zwischen 40 und 80% das Methacrolein vom Katalysator mittels Filtration kontinuierlich zu trennen und der Aufarbeitung zuzuführen.

Die erfindungsgemäße oxidative Veresterung wird in Anwesenheit des Katalysatorpartikels kontinuierlich durchgeführt. Besonders bevorzugt wird der Katalysator während der oxidativen Veresterung in einem gerührten Reaktor in Suspensionsform (als Slurry) angewendet.

## Beispiele

Beispiel 1:

Herstellung eines Katalysators mit 1,4 Gew% Au auf $TiO_2$ Träger (d50 = 20 $\mu$m).

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5M $Na_2CO_3$-Lsg. über einen Zeitraum von 10 Minuten auf 7,5 eingestellt. Anschließend werden direkt 50 g $TiO_2$ Träger (Produktname Aeroperl P25, Evonik Industries, aus $TiCl_4$ durch Flammenpyrolyse hergestellt, Korngröße d50 = 20 $\mu$m, 75% Anatas, 25% Rutil) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0.5M $Na_2CO_3$-Lsg auf 7,5 gehalten. Die Suspension wird heiß zentrifugiert, dekantiert und der Rückstand erneut und in Summe fünf Mal mit je 250 mL dest. Wasser aufgeschlämmt und erneut zentrifugiert. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest evaluiert (siehe Tabelle 1).

Beispiel 2:

Herstellung eines Katalysators mit 1,4 Gew% Au auf $TiO_2$ Träger (d50 = 40 $\mu$m)..

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine

Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest und kontinuierlich evaluiert [siehe Tabelle 1 (Batchtest) und Tabelle 2 (kontinuierlich)].

Beispiel 3:

Herstellung eines Katalysators mit 1,1 Gew% Au auf $TiO_2$ Träger (d50 = 30 $\mu$m).

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (Produktname Aeroperl P25 H6, Evonik Industries, P25 bei 600°C unter $N_2$ Atmosphäre nachkalziniert, Korngröße d50 = 30 $\mu$m) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird heiß zentrifugiert, dekantiert und der Rückstand erneut und in Summe fünf Mal mit je 250 mL dest. Wasser aufgeschlämmt und erneut zentrifugiert. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet. Der Katalysator wird unter Stickstoff auf 70 °C erwärmt und dann mit einem 5 Vol% $H_2$ in $N_2$ Strom reduziert, auf RT abgekühlt und der Gasstrom beendet. Die Performance des Katalysators wurde in einem Batchtest evaluiert (siehe Tabelle 1).

Beispiel 4:

Herstellung eines Katalysators mit 1,0 Gew% Au auf TiO2 Träger (2x5mm).

In einem Glaskolben mit mechanischer Rührung werden 1,32 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,5 und wird mit 0,5 M $Na_2CO_3$-Lsg. auf 7,5 eingestellt und dann über einen Zeitraum von 10 Minuten gehalten. Anschließend werden 50 g $TiO_2$ Träger (Aeroperl P25 mit Binder auf 2x5 mm Stäbchen extrudiert und kalziniert, Evonik Industries) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Katalysatorstäbchen werden noch heiß über eine Nutsche abfiltriert und mit dest. Wasser gewaschen bis die Restleitfähigkeit des erhaltenen Waschwassers unter 100 $\mu S/cm^2$ lag. Der erhaltene Katalysator wird in einer dünnen Schicht bei 120 °C, für 16 h getrocknet. Der Katalysator wird unter langsamer Umwälzung (6 rpm) unter Stickstoff auf 70 °C erwärmt und dann mit einem 5 Vol% $H_2$ in $N_2$ Strom reduziert, auf Raumtemperatur abgekühlt und der Gasstrom beendet.

Beispiel 5:

Herstellung eines Katalysators mit 1,0 Gew% Au auf $TiO_2$ Träger (37-100 $\mu$m).

Der Katalysator aus Beispiel 4 wurde in einem Achatmörser zerkleinert und über ein Drahtgittersieb der Anteil <100 $\mu$m abgesiebt. Der Rückstand wurde erneut im Mörser zerkleinert und die Prozedur wiederholt bis das gesamte Material auf eine Größe <100 $\mu$m zerkleinert war. Der so erhaltene Rückstand wurde über ein Sieb mit einer Maschenweite von 37 $\mu$m vom Feinanteil befreit. Der im Sieb verbleibende Katalysator mit einer Korngröße von 37-100 $\mu$m ist der Katalysator für Beispiel 5 in Tabelle 1 des Batchtests und Beispiel 2 in Tabelle 2 des kontinuierlichen Test.

Beispiel 6:

Herstellung eines Katalysators mit 1,0 Gew% Au auf $TiO_2$ Träger (<37 $\mu$m).

Der Katalysator aus Beispiel 4 wurde in einem Achatmörser zerkleinert und über ein Drahtgittersieb der Anteil <100 $\mu$m abgesiebt. Der Rückstand wurde erneut im Mörser zerkleinert und die Prozedur wiederholt bis das gesamte Material auf eine Größe <100 $\mu$m zerkleinert war. Der so erhaltene Rückstand wurde über ein Sieb mit einer Maschenweite von 37 $\mu$m vom Grobanteil befreit. Der das Sieb passierende Katalysator mit einer Korngröße von <37 $\mu$m ist der Katalysator für Beispiel 6 in Tabelle 1.

Beispiel 7:

Herstellung eines unkalzinierten Katalysators mit 1,4 Gew% Au auf $TiO_2$ Träger.

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (Produktname Aeroperl P25, Evonik Industries, aus $TiCl_4$ durch Flammenpyrolyse hergestellt, Korngröße d50 = 20 μm, 75% Anatas, 25% Rutil) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird heiß zentrifugiert, dekantiert und der Rückstand erneut und in Summe fünf Mal mit je 250 mL dest. Wasser aufgeschlämmt und erneut zentrifugiert. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet. Die Performance des so erhaltenen Katalysators wurde im Batchtest evaluiert (siehe Tabelle 1).

Beispiel 8:

Herstellung eines Katalysators mit 1,4 Gew% Au auf $TiO_2$ Träger (3h kalziniert).

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (Produktname Aeroperl P25, Evonik Industries, aus $TiCl_4$ durch Flammenpyrolyse hergestellt, Korngröße d50 = 20 μm, 75% Anatas, 25% Rutil) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2.0M $HNO_3$-Lsg. und 0,5 M $Na_2CO_3$-Lsg auf 7,5 gehalten. Die Suspension wird heiß zentrifugiert, dekantiert und der Rückstand erneut und in Summe fünf Mal mit je 250 mL dest. Wasser aufgeschlämmt und erneut zentrifugiert. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet. Der Katalysator wird unter Stickstoff auf 70 °C erwärmt und dann 3h mit einem 5 Vol% $H_2$ in $N_2$ Strom reduziert, auf Raumtemperatur abgekühlt und der Gasstrom beendet. Die Performance des Katalysators wurde in einem Batchtest evaluiert (siehe Tabelle 1).

Beispiel 9:

Herstellung eines Katalysators mit 1,4 Gew% Au auf $TiO_2$ Träger (4,5h kalziniert).

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50g $TiO_2$ Träger (Produktname Aeroperl P25, Evonik Industries, aus $TiCl_4$ durch Flammenpyrolyse hergestellt, Korngröße d50 = 20 μm, 75% Anatas, 25% Rutil) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird heiß zentrifugiert, dekantiert und der Rückstand erneut und in Summe fünf Mal mit je 250 mL dest. Wasser aufgeschlämmt und erneut zentrifugiert. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet. Der Katalysator wird unter Stickstoff auf 70 °C erwärmt und dann 4,5h mit einem 5 Vol-% $H_2$ in $N_2$ Strom reduziert, auf Raumtemperatur abgekühlt und der Gasstrom beendet. Die Performance des Katalysators wurde in einem Batchtest evaluiert (siehe Tabelle 1).

Beispiel 10:

Herstellung eines Katalysators mit 1,4 Gew% Au auf $TiO_2$ Träger (Formiat reduziert).

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 μm - Entwicklungsprodukt als stationäre Phase von HPLC Säulen, 95% Anatas Struktur)) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische

an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, für 16 h getrocknet und anschließend in einer 2 gew%ige Lösung von Natriumformiat in Methanol bei 70°C für 4h gerührt, filtriert, pH Neutral gewaschen und erneut in einer dünnen Schicht bei 120 °C für 16 h getrocknet. Die Performance des so erhaltenen Katalysators wurde im Batchtest evaluiert (siehe Tabelle 1).

Beispiel 11:

Herstellung eines Katalysators mit 1,4 Gew% Au auf $TiO_2$ Träger (Formiat reduziert in Wasser).

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur)) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C für 16 h getrocknetund anschließend in einer 2 gew%ige Lösung von Natriumformiat in Wasser bei 70°C für 4h gerührt, filtriert, pH-neutral gewaschen und erneut in einer dünnen Schicht bei 120 °C für 16 h getrocknet. Die Performance des so erhaltenen Katalysators wurde im Batchtest evaluiert (siehe Tabelle 1).

Beispiel 12:

Herstellung eines Katalysators mit 1,4 Gew% Au auf $TiO_2$ Träger (Methanol reduziert mit $O_2$).

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur)) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C, getrocknet, darauf in Methanol gerührt und für eine Stunde mit Sauerstoff begast, anschließend filtriert, pH Neutral gewaschen und erneut in einer dünnen Schicht bei 120 °C für 16 h getrocknet. Die Performance des so erhaltenen Katalysators wurde im Batchtest evaluiert (siehe Tabelle 1).

Beispiel 13:

Herstellung eines Katalysators mit 1,4 Gew% Au auf $TiO_2$ Träger (Methanol reduziert mit $N_2$).

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur)) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu S/cm^2$ liegen. Der erhaltene Katalysator wird in einer dünnen Schicht bei 120 °C für 16 h getrocknet, dann in Methanol gerührt und während dieser Zeit unter Stickstoffatmosphäre belassen, anschließend filtriert, pH Neutral gewaschen und erneut in einer dünnen Schicht bei 120 °C für 16 h getrocknet. Die Performance des so erhaltenen Katalysators wurde im Batchtest evaluiert (siehe Tabelle 1).

Beispiel 14:

Herstellung eines Katalysators mit 0,7 Gew% Au auf $TiO_2$ Träger (d50 = 40 $\mu$m).

In einem Glaskolben mit mechanischer Rührung werden 0,92 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu$S/$cm^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf RT abgekühlt und der Gasstrom beendet. Die Performance des Katalysators wurde in einem Batchtest evaluiert [siehe Tabelle 1 (Batchtest)].

Beispiel 15:

Herstellung eines Katalysators mit 0,13 Gew% Au auf $TiO_2$ Träger (d50 = 40 $\mu$m).

In einem Glaskolben mit mechanischer Rührung werden 0,19 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ Träger (TP Hombikat, Huntsman d50 = ca. 40 $\mu$m - Entwicklungsprodukt als stationäre Phase von HPLC Säulen, 95% Anatas Struktur) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu$S/$cm^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest evaluiert [siehe Tabelle 1 (Batchtest)].

Beispiel 16: Herstellung eines Katalysators Au auf $TiO_2$/$ZrO_2$ Träger.

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50g $TiO_2$/$ZrO_2$ Träger ($ZrO_2$ (15%) auf $TiO_2$ (Anatas), Huntsman d50= ca. 40 $\mu$m) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu$S/$cm^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest siehe Tabelle 1 evaluiert.

Beispiel 17: Herstellung eines Katalysators Au auf $TiO_2$/$SiO_2$ Träger.

In einem Glaskolben mit mechanischer Rührung werden 1,82 g Goldsäure ($HAuCl_4$ * 3 $H_2O$) in 600g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$/$SiO_2$ Träger ($SiO_2$(8,5%) auf $TiO_2$ (Anatas), Huntsman d50 = ca. 40 $\mu$m) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 $\mu$S/$cm^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest siehe Tabelle 1 evaluiert.

Beispiel 18: Herstellung eines Katalysators Au auf $TiO_2/Al_2O_3$ Träger.

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2/Al_2O_3$ Träger ($Al_2O_3$ (8,6%) auf $TiO_2$ (Anatas), Huntsman d50 = ca. 40 μm) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 μS/cm$^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest siehe Tabelle 1 evaluiert.

Beispiel 19: Herstellung eines Katalysators Au auf $TiO_2$ (Rutil) Träger.

In einem Glaskolben mit mechanischer Rührung werden 1,82 g Goldsäure ($HAuCl_4$ * 3 $H_2O$) in 600 g Wasser gelöst und auf 70 °C Innentemperatur erwärmt. Der pH-Wert der so erhaltenen Lösung liegt bei ca. 2,2 und wird mit 0,5 M $Na_2CO_3$-Lsg. auf 7,5 eingestellt und dann über einen Zeitraum von 10 Minuten gehalten. Anschließend werden 50 g $TiO_2$ ($TiO_2$ (Rutil), Huntsman d50 = ca. 40 μm) auf einmal unter Rühren (400 rpm) zugegeben. Die so erhaltene Suspension wird bei 70 °C für 60 min gerührt, wobei der pH-Wert konstant bei 7,5 gehalten wird. Der pH-Wert steigt während dieser Zeit ins basische an und wird mit 2,0 M $HNO_3$-Lsg. und 0,5 M $Na_2CO_3$-Lsg auf 7,5 gehalten. Die Suspension wird noch heiß über eine Nutsche mit Vakuum filtriert und 5 Mal mit je 250 mL dest. Wasser gewaschen. (5 mL dest. Wasser / g Träger). Die Restleitfähigkeit des erhaltenen Waschwassers soll unter 100 μS/cm$^2$ liegen. Der erhaltene Katalysator wird in einer dünnen Schicht bei 120 °C für 16 h getrocknet. Der Katalysator wird unter Stickstoff auf 70 °C erwärmt und dann mit einem 5 Vol% $H_2$ in $N_2$ Strom reduziert, auf Raumtemperatur abgekühlt und der Gasstrom beendet. Die Performance des Katalysators wurde in einem Batchtest siehe Tabelle 1 evaluiert.

Beispiel 20:

Vergleichsbeispiel 1 - Ni-Au-Katalysator

Gemäß der Patenliteratur (EP2210664, Bsp 1) wurde ein Nickel-Gold Katalysator hergestellt. Die Performance des Katalysators wurde in einem Batchtest siehe Tabelle 1 und kontinuierlich siehe Tabelle 2 evaluiert.

Beispiel 21:

Vergleichsbeispiel 2 - Co-Au-Katalysator

Gemäß der Patenliteratur (WO2017084969, Bsp 4) wurde ein Cobalt-Gold Katalysator hergestellt. Performance des Katalysators wurde in einem Batchtest siehe Tabelle 1 und kontinuierlich siehe Tabelle 2 evaluiert.

Beispiel 22:

Die Ansatzgröße aus Beispiel 2 wurde um den Faktor sieben skaliert und insgesamt dreimal mit 350 g eingesetzten Träger reproduziert. Die Aktivität und Selektivität der einzelnen Reproduktionen ergab - auch im Vergleich mit Beispiel 2 - keinerlei Streuung die über die Messgenauigkeitsgrenze hinausging. Die so erhaltenen Katalysatorcargen wurden vereinigt, gemischt und von dieser Mischung 600 g entnommen, welche in einem 20L Kessel vorgelegt wurde.

Methacrolein wird mit einer konstanten Rate von 500g/h kontinuierlich in den gerührten und von unten begasten 20 L Rührkesselreaktor (Begasung mit Luft bis zu einem Restsauerstoffgehalt von bis zu 4 Vol% im Abgas.) unter 5 bar Druck und 80 °C Innentemperatur zugeführt. Pro Mol zugeführtes Methacrolein wurden gleichzeitig 4 Mol Methanol dem Reaktor zugeführt und dieses Verhältnis stets konstant gehalten. Gleichzeitig wurde in diesen Reaktor so viel NaOH-Lösung (in Methanol/$H_2O$ (95:5) zugeführt, dass der Wert pH = 7 im Reaktor konstant blieb. Das Reaktionsgemisch wurde über einen Filter aus dem Reaktor kontinuierlich entnommen. Die Produktproben wurden nach der in Tabelle 4 angegebenen Zeit entnommen und mittels GC und HPLC analysiert. Insbesondere der Wert für Methacrylsäure lässt sich so besonders präzise, auch in Form von dessen in situ gebildetem Natriumsalz bestimmen. Die Bildung von Isobuttersäuremethylester fällt innerhalb der ersten zwei Tage sehr stark ab und erreicht nach etwa vier Tagen einen stationären Wert von 50 ppm, der über die gesamte Versuchslaufzeit weitgehend konstant verbleibt. Die Aktivität ist

durchgehend stabil und hoch. Tabelle 4 und Abbildung 1 bis 4.

Batchtests zur MMA-Herstellung (Anleitung zur Tabelle 1)

Ein Gold-haltiger Katalysator gemäß einem der Beispiele 1 bis 21 (384 mg), Methacrolein (1,20 g) und Methanol (9,48g) wurden 2h bei 60 °C Innentemperatur und 30 bar Druck in einer Atmosphäre von 7 Vol% $0_2$ in $N_2$ in einem 140 ml Stahlautoklaven mit Magnetrührer gerührt. Nach 2h wurde das Gemisch abgekühlt, entgast, filtriert und mittels GC analysiert. Jeder Katalysator wurde mindestens zweimal unter identischen Bedingungen getestet, die Ergebnisse der jeweiligen Experimente gemittelt. Der resultierenden Umsatz von Methacrolein (U(MAL), in %), und die Selektivität zu MMA (S(MMA), in %) für jeden getesteten Katalysator sind in der folgenden Tabelle 1 zusammengefasst.

Kontinuierlicher Test zur Herstellung von MMA (Allgemeine Beschreibung zur Tabelle 2)

Der pH-Wert einer 42,5 gew%igen Lösung von MAL in Methanol wird durch die Zugabe von einer 1 gew%igen Lösung NaOH in Methanol unter Rühren auf pH = 7 eingestellt. Diese Lösung wird mit einer konstanten Zugaberate kontinuierlich einem gerührten und begasten Rührkesselreaktor (Begasung mit Luft) unter 10 bar Druck und 80 °C Innentemperatur zugeführt. Gleichzeitig werden in diesen Reaktor mit 20 g Pulverkalalysator (aus einem vorherigem Beispiel) so viel 1 gew%ige NaOH-Lösung (in Methanol) zugeführt, dass der Wert pH = 7 im Reaktor konstant blieb. Das Reaktionsgemisch wurde über einen Filter aus dem Reaktor kontinuierlich entnommen. Die Produktproben wurden nach der in Tabelle 2 angegebener Zeit entnommen und mittels GC analysiert.

Tabelle 1: Katalysatorevaluation durch Batchtest

| | Bsp. Nr | Katalysator | Variation | Umsatz [MAL] | Selektivität [MMA] |
|---|---|---|---|---|---|
| Katalysatorkorn | 1 | 1,4% Au auf TiO2 | P25, Evonik Industries | 93,1% | 94,9% |
| | 2 | 1,4% Au auf TiO2 | Hombikat, Huntsman | 91,7% | 90,9% |
| | 3 | 1,1% Au auf TiO2 | P25 H6, Evonik Industries (nachkalziniert) | 95,3% | 91,0% |
| | 4 | 1% Au auf TiO2 | Granuliert & nachkalziniert --> ungemörsert 2x5mm | 75,1% | 93,2% |
| | 5 | 1% Au auf TiO2 | gemörsert und gesiebt auf 36-100µm | 87,8% | 93,0% |
| | 6 | 1% Au auf TiO2 | gemörsert und gesiebt auf <36µm | 88,5% | 92,7% |
| | Bsp. Nr | Katalysator | | Umsatz [MAL] | Selektivität [MMA] |
| Kalzinierungsmethode | 7 | 1,4% Au auf TiO2 | Unkalziniert | 33,1% | 96,6% |
| | 8 | 1,4% Au auf TiO2 | 3h, 70°C, N2, H2 Strom kalziniert | 92,7% | 93,0% |
| | 9 | 1,4% Au auf TiO2 | 4,5h, 70°C, N2, H2 Strom kalziniert | 93,2% | 91,1% |
| | 10 | 1,4% Au auf TiO2 | 2 gew% Formiat in Methanol | 68,4% | 89,1% |
| | 11 | 1,4% Au auf TiO2 | 2 gew% Formiat in Wasser | | |
| | 12 | 1,4% Au auf TiO2 | 1h, in MeOH mit O2 | 96,1% | 93,4% |
| | 13 | 1,4% Au auf TiO2 | 1h, in MeOH mit N2 | | |
| | Bsp. Nr | | | Umsatz [MAL] | Selektivität [MMA] |
| Gold-menge | 14 | 0,7% Au auf TiO2 | Hombikat, Huntsman | 70,2% | 87,9% |
| | 15 | 0,1% Au auf TiO2 | Hombikat, Huntsman | 18,4% | 58,7% |
| | Bsp. Nr | | | Umsatz [MAL] | Selektivität [MMA] |
| Mischträger | 16 | Au auf TiO2/ZrO2 | Huntsman, Träger | 65,9% | 81,1% |
| | 17 | Au auf TiO2/SiO2 | Huntsman, Träger | | |
| | 18 | Au auf TiO2/Al2O3 | Huntsman, Träger | | |
| | 19 | Au auf TiO2 (Rutil) | Huntsman, Träger | | |
| | Bsp. Nr | | | Umsatz [MAL] | Selektivität [MMA] |
| Vergleich | 20 | NiAu auf SiO/Al2O3/MgO | EP2210664, Bsp 1 | 81,3% | 93,2% |
| | 21 | CoAu auf SiO/Al2O3/MgO | WO2017084969, Bsp 4 | 80,1% | 93,2% |

Tabelle 2: Katalysatorevaluation durch kontinuierliche Testung

| Kat. Aus Bsp. Nr | Katalysator | TOS [h] | Umsatz %[MAL] | Selektivität %[MMA] | Selektivität % [hydriertes MMA] | Batchtest Nr (Referenz) |
|---|---|---|---|---|---|---|
| | | 21 | 77,1 | 92,1 | 0,05 | |
| 2 | 1,4% Au auf TiO2 | 495 | 77,89 | 92,4 | 0,01 | 2 |
| | | 999 | 78,5 | 91,3 | 0,01 | |

(fortgesetzt)

| Kat. Aus Bsp. Nr | Katalysator | TOS [h] | Umsatz %[MAL] | Selektivität %[MMA] | Selektivität % [hydriertes MMA] | Batchtest Nr (Referenz) |
|---|---|---|---|---|---|---|
| 5 | 1,0% Au auf TiO2, gemörsert | 28 | 85,1 | 92,2 | 0,06 | 5 |
| | | 77 | 83,1 | 92,1 | 0,01 | |
| | | 240 | 78,2 | 92 | 0,01 | |
| | | 500 | 71,3 | 92,1 | 0,01 | |
| 20 | NiAu-Katalysator | 22 | 75,1 | 92 | 0,01 | 20 |
| | | 525 | 74,8 | 91,9 | 0,13 | |
| | | 676 | 74,6 | 92,1 | 0,12 | |
| | | 1000 | 74,1 | 91,9 | 0,13 | |
| 21 | CoAu-Katalysator | 50 | 73,5 | 92,3 | 0,02 | 21 |
| | | 200 | 71,09 | 91,8 | 0,08 | |
| | | 380 | 70,8 | 91,5 | 0,09 | |
| | | 480 | 70,6 | 92,1 | 0,08 | |

Kontinuierlicher Test zur Herstellung von MMA bei variiertem pH-Wert (Beschreibung Tabelle 3) Der pH-Wert einer 42,5 gew%igen Lösung von MAL in Methanol wird durch die Zugabe von einer 1 gew%igen Lösung NaOH in Methanol unter Rühren auf pH = 7 eingestellt. Diese Lösung wird mit einer konstanten Zugaberate kontinuierlich einem gerührten und begasten Rührkesselreaktor (Begasung mit Luft) unter 10 bar Druck und 80 °C Innentemperatur zugeführt. Gleichzeitig werden in diesen Reaktor mit 20 g Pulverkatalysator aus Beispiel 2 so viel 1 gew%ige NaOH-Lösung (in Methanol) zugeführt, dass der pH im Reaktor auf den in Tabelle 3 erwähnten Wert konstant eingestellt blieb. Das Reaktionsgemisch wurde über einen Filter aus dem Reaktor kontinuierlich entnommen. Es wurden jeweils mindestens drei Produktproben (mit 24h Wartezeit) entnommen und mittels GC analysiert, bevor der pH Wert variiert wurde.

**Tabelle 3:**

| Kat. Aus Bsp. Nr | Katalysator | **pH-Wert** | **Umsatz %[MAL]** | Selektivität %[MMA] | Selektivität % [DiMAL & DiMAL-Ester] | Selektivität % [Dimethoxyisobuten] | Selektivität % [3-MIBSM] |
|---|---|---|---|---|---|---|---|
| 2 | 1,4% Au auf $TiO_2$ | **6,6** | **78,7%** | 91,7% | 0,248% | 0,228% | 0,792% |
| | | **7,0** | **86,3%** | 92,4% | 0,084% | 0,064% | 0,813% |
| | | **7,6** | **92,2%** | **93,0%** | 0,037% | 0,017% | 0,879% |
| | | **8,0** | **96,8%** | 91,9% | 0,011% | 0,007% | 0,912% |
| | | **8,3** | **98,1%** | 92,1% | 0,012% | 0,001% | 0,961% |

Tabelle 4: Kontinuierliche Reaktionsführung im 20L Druckreaktor.

| hr | % | Wt.% | Wt.% | Wt.% | Wt.% | Wt.% | Wt.% | Wt.% | Wt.% | Wt.% | hr | mol MAL /hr.kg.Cat | mol MMA /hr.kg.Cat | Mol /mol | Mol/mol | mol/mol |
|---|---|---|---|---|---|---|---|---|---|---|---|---|---|---|---|---|
| TOS | MAL Conver -sion | Hydrogenate d MMA | MMA | 2-HibsM | Dimethox yisobuten | MAS | 3-MibsM | 3-HibsM | DiMAL-Ester | Sum C4 Se-lectivities | Reis-dence Time | WHSV | RZA | MeOH/ MAL in feed | Me-OH/MAL in Reactor | H2O/MAL im reactor |
| 14,75 | 82,32% | 0,29% | 73,64% | 0,09% | 0,11% | 2,12% | 1,14% | 0,00% | 0,00% | 77,16% | 8,74 | 11,51 | 7,11 | 4,05 | 22,31 | 4,81 |
| 22,75 | 77,75% | 0,14% | 87,18% | 0,10% | 0,15% | 3,01% | 1,38% | 0,00% | 0,00% | 91,82% | 8,78 | 11,52 | 8,12 | 4,05 | 15,77 | 3,63 |
| 30,75 | 75,13% | 0,09% | 92,06% | 0,10% | 0,19% | 3,82% | 1,62% | 0,00% | 0,00% | 97,82% | 8,77 | 11,50 | 8,31 | 4,06 | 13,50 | 3,15 |
| 38,75 | 75,00% | 0,07% | 92,85% | 0,09% | 0,21% | 4,33% | 1,75% | 0,00% | 0,02% | 99,32% | 8,77 | 11,52 | 8,39 | 4,05 | 13,47 | 3,32 |
| 46,75 | 74,34% | 0,06% | 93,32% | 0,09% | 0,22% | 4,28% | 1,82% | 0,09% | 0,01% | 99,89% | 8,78 | 11,51 | 8,34 | 4,06 | 13,22 | 3,27 |
| 54,75 | 74,36% | 0,05% | 93,40% | 0,09% | 0,21% | 4,21% | 1,82% | 0,00% | 0,04% | 99,82% | 8,78 | 11,50 | 8,35 | 4,05 | 12,61 | 2,89 |
| 62,75 | 74,55% | 0,03% | 93,72% | 0,08% | 0,22% | 4,30% | 1,87% | 0,00% | 0,03% | 100,25% | 8,78 | 11,48 | 8,39 | 4,06 | 13,15 | 2,96 |
| 70,75 | 74,60% | 0,05% | 93,39% | 0,08% | 0,21% | 4,29% | 1,84% | 0,00% | 0,05% | 99,90% | 8,78 | 11,52 | 8,39 | 4,05 | 12,95 | 3,01 |
| 78,75 | 74,37% | 0,04% | 92,89% | 0,08% | 0,20% | 4,13% | 1,83% | 0,04% | 0,24% | 99,41% | 8,78 | 11,55 | 8,33 | 4,04 | 13,07 | 3,43 |
| 86,75 | 74,25% | 0,04% | 93,19% | 0,07% | 0,19% | 4,36% | 1,85% | 0,04% | 0,25% | 99,98% | 8,78 | 11,55 | 8,34 | 4,05 | 13,06 | 3,52 |
| 94,75 | 74,24% | 0,04% | 93,40% | 0,07% | 0,19% | 4,42% | 1,84% | 0,03% | 0,30% | 100,28% | 8,78 | 11,51 | 8,34 | 4,06 | 13,14 | 3,50 |
| 102,75 | 74,26% | 0,04% | 92,84% | 0,07% | 0,19% | 4,24% | 1,81% | 0,04% | 0,26% | 99,49% | 8,77 | 11,55 | 8,31 | 4,05 | 13,10 | 3,51 |
| 110,75 | 74,45% | 0,04% | 92,76% | 0,07% | 0,19% | 4,25% | 1,83% | 0,03% | 0,28% | 99,45% | 8,78 | 11,55 | 8,33 | 4,05 | 13,19 | 3,58 |
| 118,75 | 74,15% | 0,04% | 93,49% | 0,07% | 0,19% | 4,45% | 1,85% | 0,03% | 0,27% | 100,39% | 8,78 | 11,51 | 8,34 | 4,06 | 13,11 | 3,53 |
| 126,75 | 74,12% | 0,04% | 92,53% | 0,07% | 0,19% | 4,37% | 1,85% | 0,04% | 0,28% | 99,37% | 8,77 | 11,55 | 8,26 | 4,05 | 13,02 | 3,41 |
| 134,75 | 74,21% | 0,04% | 92,82% | 0,07% | 0,19% | 4,20% | 1,86% | 0,04% | 0,27% | 99,49% | 8,78 | 11,55 | 8,29 | 4,05 | 13,08 | 3,55 |
| 142,75 | 74,15% | 0,03% | 93,20% | 0,07% | 0,19% | 4,35% | 1,85% | 0,05% | 0,26% | 99,99% | 8,78 | 11,53 | 8,31 | 4,05 | 12,88 | 3,57 |
| 150,75 | 74,19% | 0,03% | 92,67% | 0,07% | 0,19% | 4,30% | 1,83% | 0,03% | 0,26% | 99,40% | 8,78 | 11,55 | 8,25 | 4,05 | 12,86 | 3,39 |
| 158,75 | 74,11% | 0,03% | 92,99% | 0,07% | 0,19% | 4,26% | 1,82% | 0,04% | 0,26% | 99,67% | 8,78 | 11,54 | 8,27 | 4,05 | 12,92 | 3,36 |
| 166,75 | 74,15% | 0,03% | 93,15% | 0,07% | 0,20% | 4,29% | 1,83% | 0,04% | 0,28% | 99,86% | 8,78 | 11,53 | 8,30 | 4,05 | 13,06 | 3,39 |
| 174,75 | 74,18% | 0,03% | 92,55% | 0,07% | 0,20% | 4,33% | 1,82% | 0,05% | 0,26% | 99,31% | 8,78 | 11,55 | 8,22 | 4,05 | 12,87 | 3,36 |

(fortgesetzt)

**Tabelle 4: Fortsetzung 182h - 358h Laufzeit**

| hr | % | Wt.% | Wt.% | Wt.% | Wt.% | Wt.% | Wt.% | Wt.% | Wt.% | Wt.% | hr | mol MAL /hr.kg.Cat | mol MMA /hr.kg.Cat | Mol /mol | Mol/mol | mol/mol |
|---|---|---|---|---|---|---|---|---|---|---|---|---|---|---|---|---|
| TOS | MAL Conver -sion | Hydrogenate d MMA | MMA | 2-HibsM | Dimethox yisobuten | MAS | 3-MibsM | 3-HibsM | DiMAL-Ester | Sum C4 Se-lectivities | Reis-dence Time | WHSV | RZA | MeOH/ MAL in feed | Me-OH/MAL in Reactor | H2O/MAL im reactor |
| 182,75 | 74,21% | 0,03% | 93,17% | 0,07% | 0,20% | 4,52% | 1,84% | 0,04% | 0,26% | 100,11% | 8,78 | 11,54 | 8,27 | 4,05 | 12,95 | 3,45 |
| 190,75 | 74,25% | 0,04% | 93,14% | 0,07% | 0,20% | 4,34% | 1,84% | 0,05% | 0,28% | 99,93% | 8,78 | 11,56 | 8,28 | 4,05 | 12,90 | 3,36 |
| 198,75 | 74,18% | 0,04% | 92,66% | 0,07% | 0,20% | 4,53% | 1,86% | 0,05% | 0,27% | 99,66% | 8,78 | 11,54 | 8,19 | 4,05 | 12,80 | 3,42 |
| 206,75 | 74,21% | 0,04% | 92,85% | 0,07% | 0,20% | 4,28% | 1,86% | 0,04% | 0,28% | 99,58% | 8,78 | 11,55 | 8,18 | 4,05 | 12,66 | 3,34 |
| 214,75 | 74,19% | 0,04% | 93,01% | 0,07% | 0,20% | 4,31% | 1,84% | 0,05% | 0,28% | 99,79% | 8,77 | 11,55 | 8,24 | 4,05 | 12,87 | 3,51 |
| 222,75 | 74,18% | 0,04% | 93,17% | 0,07% | 0,20% | 4,44% | 1,87% | 0,05% | 0,28% | 100,12% | 8,78 | 11,51 | 8,18 | 4,05 | 12,74 | 3,38 |
| 230,75 | 74,10% | 0,04% | 93,40% | 0,07% | 0,20% | 4,47% | 1,87% | 0,05% | 0,29% | 100,38% | 8,78 | 11,50 | 8,18 | 4,06 | 12,64 | 3,35 |
| 239,25 | 74,18% | 0,04% | 93,25% | 0,07% | 0,20% | 4,37% | 1,86% | 0,05% | 0,27% | 100,06% | 8,78 | 11,54 | 8,24 | 4,04 | 12,72 | 3,29 |
| 246,75 | 74,24% | 0,04% | 92,68% | 0,07% | 0,20% | 4,37% | 1,87% | 0,04% | 0,28% | 99,50% | 8,78 | 11,55 | 8,12 | 4,04 | 12,49 | 3,31 |
| 254,75 | 74,23% | 0,04% | 93,39% | 0,07% | 0,20% | 4,42% | 1,87% | 0,04% | 0,30% | 100,30% | 8,78 | 11,52 | 8,15 | 4,06 | 12,47 | 3,26 |
| 262,75 | 74,12% | 0,04% | 93,92% | 0,07% | 0,20% | 4,42% | 1,87% | 0,04% | 0,24% | 100,77% | 8,78 | 11,51 | 8,21 | 4,06 | 12,55 | 3,25 |
| 270,75 | 74,31% | 0,04% | 92,63% | 0,07% | 0,20% | 4,37% | 1,88% | 0,04% | 0,25% | 99,45% | 8,78 | 11,52 | 8,06 | 4,05 | 12,66 | 3,23 |
| 278,75 | 74,24% | 0,04% | 93,20% | 0,07% | 0,20% | 4,43% | 1,90% | 0,04% | 0,29% | 100,14% | 8,78 | 11,49 | 8,08 | 4,06 | 12,40 | 3,22 |
| 286,75 | 74,19% | 0,04% | 93,43% | 0,07% | 0,20% | 4,44% | 1,89% | 0,04% | 0,28% | 100,35% | 8,79 | 11,48 | 8,12 | 4,06 | 12,47 | 3,28 |
| 294,75 | 74,11% | 0,04% | 92,81% | 0,07% | 0,20% | 4,46% | 1,88% | 0,04% | 0,28% | 99,75% | 8,78 | 11,53 | 8,05 | 4,04 | 12,74 | 3,21 |
| 302,75 | 74,15% | 0,04% | 92,97% | 0,07% | 0,21% | 4,37% | 1,88% | 0,04% | 0,28% | 99,81% | 8,78 | 11,52 | 8,05 | 4,05 | 12,50 | 3,27 |
| 326,75 | 74,27% | 0,04% | 93,33% | 0,07% | 0,21% | 4,45% | 1,90% | 0,04% | 0,27% | 100,29% | 8,78 | 11,55 | 8,08 | 4,03 | 12,71 | 3,76 |
| 334,75 | 74,24% | 0,04% | 93,24% | 0,07% | 0,22% | 4,50% | 1,90% | 0,04% | 0,29% | 100,30% | 8,79 | 11,56 | 8,11 | 4,03 | 12,54 | 3,65 |
| 342,75 | 74,30% | 0,04% | 93,06% | 0,07% | 0,22% | 4,76% | 1,88% | 0,03% | 0,29% | 100,41% | 8,78 | 11,56 | 8,09 | 4,03 | 12,50 | 3,67 |
| 350,75 | 74,24% | 0,04% | 93,00% | 0,07% | 0,21% | 4,61% | 1,93% | 0,04% | 0,29% | 100,24% | 8,78 | 11,55 | 8,09 | 4,03 | 12,73 | 3,66 |
| 358,75 | 74,24% | 0,04% | 93,17% | 0,07% | 0,22% | 4,59% | 1,91% | 0,04% | 0,29% | 100,45% | 8,78 | 11,53 | 8,05 | 4,04 | 12,62 | 3,66 |

(fortgesetzt)

**Tabelle 4: Fortsetzung 382h - 422h Laufzeit**

| hr | % | Wt.% | Wt.% | Wt.% | Wt.% | Wt.% | Wt.% | Wt.% | Wt.% | Wt.% | hr | mol MAL /hr.kg.Cat | mol MMA /hr.kg.Cat | Mol /mol | Mol/mol | mol/mol |
|---|---|---|---|---|---|---|---|---|---|---|---|---|---|---|---|---|
| TOS | MAL Conver -sion | Hydrogenate d MMA | MMA | 2-HibsM | Dimethox yisobuten | MAS | 3-MibsM | 3-HibsM | DiMAL-Ester | Summe C4 Selektivität | Ver-weilzeit | WHSV | RZA | MeOH/ MALim Feed | Me-OH/MAL in Reaktor | H2O/MAL im Reaktor |
| 382,75 | 74,15% | 0,04% | 91,52% | 0,07% | 0,22% | 4,52% | 1,94% | 0,03% | 0,27% | 98,04% | 8,78 | 11,52 | 8,00 | 4,05 | 12,82 | 3,47 |
| 390,75 | 74,33% | 0,04% | 93,16% | 0,07% | 0,21% | 4,32% | 1,90% | 0,04% | 0,26% | 99,96% | 8,78 | 11,50 | 8,04 | 4,07 | 12,53 | 3,53 |
| 398,75 | 74,25% | 0,04% | 93,25% | 0,07% | 0,22% | 4,51% | 1,93% | 0,04% | 0,29% | 100,24% | 8,78 | 11,50 | 8,01 | 4,05 | 12,63 | 3,52 |
| 406,75 | 74,33% | 0,04% | 93,88% | 0,07% | 0,23% | 4,38% | 1,93% | 0,04% | 0,30% | 100,76% | 8,78 | 11,52 | 8,04 | 4,04 | 12,67 | 3,46 |
| 414,75 | 74,15% | 0,04% | 93,26% | 0,07% | 0,24% | 4,52% | 1,89% | 0,03% | 0,31% | 100,83% | 8,78 | 11,51 | 7,96 | 4,04 | 12,73 | 3,44 |
| 422,75 | 74,19% | 0,04% | 93,35% | 0,07% | 0,24% | 4,66% | 1,94% | 0,03% | 0,31% | 100,62% | 8,79 | 11,51 | 7,97 | 4,04 | 12,65 | 3,49 |

Erläuterung:

TOS =Laufzeit (time on stream); MAL Conversion =Umsatz von Methacrolein; Hydrogenated MMA = Isobuttersäuremethylester; MMA = Methylmethacrylat; 2-HibsM = 2-Hydroxyisobuttersäuremethylester; MAS = Methacrylsäure; 3-MibsM = 3-Methoxyisobuttersäuremethylester; 3-HibsM = 3-Hydroxyisobuttersäuremethylester;

Di-MAL-Ester =

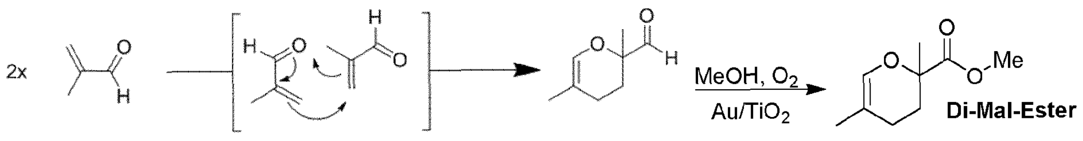


Diels-Alder-Dimerisierungsprodukt von Methacrolein nach Direktoxidation vom Aldehyd zum Ester.

**Zu den Abbildungen:**

Abbildung 1 zeigt zu Tabelle 4 den Methacroleinumsatz in Abhängigkeit von der Laufzeit im kontinuierlichen Betrieb. Damit lässt sich insbesondere die Langzeitaktivität des Katalysators belegen.

Abbildung 2 zeigt zu Tabelle 4 die Abhängigkeit der Bildung des Nebenproduktes hydriertes MMA in Abhängigkeit von der Versuchslaufzeit im kontinuierlichen Betrieb.

Abbildung 3 zeigt zu Tabelle 4 die Zusammensetzung des Reaktorabstroms für Komponenten, die in einer Konzentration von größer 1 Gew% vorliegen, im kontinuierlichen Betrieb.

Abbildung 4 zeigt zu Tabelle 4 die Zusammensetzung des Reaktorabstroms für Komponenten, die in einer Konzentration kleiner 1 Gew% vorliegen, im kontinuierlichen Betrieb.

**Patentansprüche**

1. Verfahren zur oxidativen Veresterung eines Aldehyds zu einem Carbonsäureester in Anwesenheit eines Katalysatorpartikels, **dadurch gekennzeichnet, dass** der Partikel aus 0,1 bis 3 Gew% Gold, 25 bis 99,8 Gew% $TiO_2$, 0 bis 50 Gew% Siliziumoxiden, 0 bis 25 Gew% $Al_2O_3$, 0 bis 25 Gew% Oxiden von Alkalimetallen, Erdalkalimetallen Seltenerdemetallen und/oder Zirkonium, 0 bis 20 Gew% Zirkonium-, Eisen-, Zink-, Nickel- und/oder Kobaltoxiden und 0 bis 5 Gew% weiteren Komponenten besteht, dass das Gold, optional zusammen mit Eisen-, Zink-, Nickel- und/oder Kobaltoxiden in Form von Partikeln mit einem mittleren Durchmesser zwischen 2 und 10 nm, gebunden an den Katalysatorpartikel vorliegt, dass der Katalysatorpartikel einen mittleren geometrischen Äquivalenzdurchmesser zwischen 5 $\mu$m und 10 mm aufweist, und dass das Verfahren kontinuierlich, mittels einer heterogenen Katalyse in einer Flüssigphase durchgeführt wird.

2. Verfahren gemäß Anspruch 1, **dadurch gekennzeichnet, dass** der Katalysatorpartikel aus 0,3 bis 2,5 Gew% Gold, 50 bis 99,5 Gew% $TiO_2$, 0 bis 20 Gew% Siliziumoxiden, 0 bis 10 Gew% Eisen-, Zink-, Nickel- und/oder Kobaltoxiden und 0 bis 5 Gew% weiteren Komponenten besteht.

3. Verfahren gemäß Anspruch 1 oder 2, **dadurch gekennzeichnet, dass** der Katalysatorpartikel aus 0,3 bis 2,5 Gew% Gold, 96 bis 99,5 Gew% $TiO_2$ und maximal 3,7 Gew% weiterer Komponenten, bei denen es sich weder um Gold noch um $TiO_2$ handelt, besteht.

4. Verfahren gemäß mindestens einem der Ansprüche 1 bis 3, **dadurch gekennzeichnet, dass** das Gold und/oder Gold- und Eisen-, Zink-, Nickel- und/oder Kobaltoxid-haltige Partikel mit einem mittleren Durchmesser zwischen 2 und 10 nm im Außenbereich des Katalysatorpartikels vorliegen.

5. Verfahren gemäß mindestens einem der Ansprüche 1 bis 3, **dadurch gekennzeichnet, dass** das Gold und/oder Gold- und Eisen-, Zink-, Nickel- und/oder Kobaltoxid-haltige Partikel mit einem mittleren Durchmesser zwischen 2 und 10 nm gleichmäßig über den Katalysatorpartikel verteilt vorliegen.

6. Verfahren gemäß mindestens einem der Ansprüche 1 bis 5, **dadurch gekennzeichnet, dass** die Katalysatorpartikel porös sind, eine spezifische Oberfläche zwischen 20 und 300 $m^2/g$ aufweisen, und dass deren durchschnittlicher Porendurchmesser 2 bis 30 nm beträgt.

7. Verfahren gemäß Anspruch 6, **dadurch gekennzeichnet, dass** die spezifische Oberfläche des Katalysatorpartikels zwischen 30 und 200 $m^2/g$ liegt, und dass der durchschnittliche Porendurchmesser 5 bis 25 nm beträgt.

8. Verfahren gemäß mindestens einem der Ansprüche 1 bis 7, **dadurch gekennzeichnet, dass** es sich bei dem Verfahren um eine oxidative Veresterung eines Aldehyds in Gegenwart von Sauerstoff und einem Alkohol zu einem Carbonsäureester handelt.

9. Verfahren gemäß Anspruch 8, **dadurch gekennzeichnet, dass** es sich bei dem Verfahren um die oxidative Veresterung von Methacrolein in Gegenwart von Sauerstoff und einem Alkohol zu einem Alkylmethacrylat.

10. Verfahren gemäß Anspruch 1 und 9, **dadurch gekennzeichnet, dass** die Reaktion zwischen Methacrolein und Methanol in Gegenwart eines sauerstoffhaltigen Gases unter Einhaltung der folgenden Parameter kontinuierlich durchgeführt wird:

    I. bei einer Reaktionstemperatur zwischen 40-120°C,
    II. bei einem absoluten Druck zwischen 1 und 20 bar,
    III. mit einer Slurry Dichte des Katalysators, der in der stationären Produktmischung, enthaltend MMA, Methacrolein, Methanol und Wasser, gerührt und verwirbelt vorliegt, zwischen 1 und 20 Gew%,

    wobei das Edukt Methacrolein in jeweils mindestens einem Reaktor nicht vollständig umgesetzt wird, sondern nach einem Partialumsatz zwischen 20 und 95% vom Katalysator mittels Filtration kontinuierlich abgetrennt und der Aufarbeitung zugeführt wird.

11. Verfahren gemäß Anspruch 10, **dadurch gekennzeichnet, dass** Luft als sauerstoffhaltiges Gas kontinuierlich dem Reaktor zugeführt wird, wobei der Sauerstoffgehalt der sich über der Reaktionsmischung bildenden und befindlichen Gasphase zwischen 2 und 8 Vol% beträgt.

12. Verfahren gemäß Anspruch 10 oder 11, **dadurch gekennzeichnet, dass** die Reaktion bei einer Reaktionstemperatur zwischen 50 und 100°C bei einem absoluten Druck zwischen 2 und 15 bar betrieben wird.

13. Verfahren gemäß mindestens einem der Ansprüche 10 bis 12, **dadurch gekennzeichnet, dass** die Slurry Dichte des partikulären Katalysators zwischen 2 und 15 Gew% beträgt.

14. Verfahren gemäß mindestens einem der Ansprüche 10 bis 13, **dadurch gekennzeichnet, dass** das Edukt Methacrolein in jeweils einem Reaktor oder mehreren in Reihe geschalteten Reaktoren nicht vollständig umgesetzt wird, sondern nach einem Partialumsatz zwischen 40 und 80% vom Katalysator mittels Filtration kontinuierlich abgetrennt und der Aufarbeitung zugeführt wird.

## Claims

1. Process for oxidative esterification of an aldehyde to a carboxylic ester in the presence of a catalyst particle, **characterized in that** the particle consists of 0.1% to 3% by weight of gold, 25% to 99.8% by weight of $TiO_2$, 0% to 50% by weight of silicon oxides, 0% to 25% by weight of $Al_2O_3$, 0% to 25% by weight of oxides of alkali metals, alkaline earth metals, rare earth metals and/or zirconium, 0% to 20% by weight of zirconium oxides, iron oxides, zinc oxides, nickel oxides and/or cobalt oxides and 0% to 5% by weight of further components, **in that** the gold, optionally together with iron oxides, zinc oxides, nickel oxides and/or cobalt oxides, is present bound to the catalyst particle in the form of particles having an average diameter between 2 and 10 nm, **in that** the catalyst particle has an average geometric equivalent diameter between 5 μm and 10 mm, and **in that** the process is conducted continuously, by means of heterogeneous catalysis in a liquid phase.

2. Process according to Claim 1, **characterized in that** the catalyst particle consists of 0.3% to 2.5% by weight of gold, 50% to 99.5% by weight of $TiO_2$, 0% to 20% by weight of silicon oxides, 0% to 10% by weight of iron oxides, zinc oxides, nickel oxides and/or cobalt oxides and 0% to 5% by weight of further components.

3. Process according to Claim 1 or 2, **characterized in that** the catalyst particle consists of 0.3% to 2.5% by weight of gold, 96% to 99.5% by weight of $TiO_2$ and not more than 3.7% by weight of further components which are neither gold nor $TiO_2$.

4. Process according to at least one of Claims 1 to 3, **characterized in that** the gold and/or gold- and iron oxide-, zinc oxide-, nickel oxide- and/or cobalt oxide-containing particles having an average diameter between 2 and 10 nm are present in the outer region of the catalyst particle.

5. Process according to at least one of Claims 1 to 3, **characterized in that** the gold and/or gold- and iron oxide-, zinc oxide-, nickel oxide- and/or cobalt oxide-containing particles having an average diameter between 2 and 10 nm are distributed homogeneously across the catalyst particle.

6. Process according to at least one of Claims 1 to 5, **characterized in that** the catalyst particles are porous and have a specific surface area between 20 and 300 $m^2/g$, and **in that** the average pore diameter thereof is 2 to 30 nm.

7. Process according to Claim 6, **characterized in that** the specific surface area of the catalyst particle is between 30 and 200 $m^2/g$, and **in that** the average pore diameter is 5 to 25 nm.

8. Process according to at least one of Claims 1 to 7, **characterized in that** the process is an oxidative esterification of an aldehyde in the presence of oxygen and an alcohol to give a carboxylic ester.

9. Process according to Claim 8, **characterized in that** the process is the oxidative esterification of methacrolein in the presence of oxygen and an alcohol to give an alkyl methacrylate.

10. Process according to Claims 1 and 9, **characterized in that** the reaction between methacrolein and methanol is conducted continuously in the presence of an oxygenous gas observing the following parameters:

    I. at a reaction temperature between 40-120°C,
    II. at an absolute pressure between 1 and 20 bar,
    III. with a slurry density of the catalyst, which is being stirred and swirled in the steady-state product mixture comprising MMA, methacrolein, methanol and water, between 1% and 20% by weight,

    wherein the methacrolein reactant is not fully converted in at least one reactor in each case, but after a partial conversion between 20% and 95% is separated continuously from the catalyst by means of filtration and fed to the workup.

11. Process according to Claim 10, **characterized in that** air is supplied continuously to the reactor as oxygenous gas, where the oxygen content of the gas phase present that forms above the reaction mixture is between 2% and 8% by volume.

12. Process according to Claim 10 or 11, **characterized in that** the reaction is conducted at a reaction temperature between 50°C and 100°C at an absolute pressure between 2 and 15 bar.

13. Process according to at least one of Claims 10 to 12, **characterized in that** the slurry density of the particulate catalyst is between 2% and 15% by weight.

14. Process according to at least one of Claims 10 to 13, **characterized in that** the methacrolein reactant is not fully converted in one reactor in each case or multiple reactors connected in series, but after a partial conversion between 40% and 80% is separated continuously from the catalyst by means of filtration and fed to the workup.

**Revendications**

1. Procédé d'estérification oxydative d'un aldéhyde en un ester d'acide carboxylique en présence d'une particule de catalyseur, **caractérisé en ce que** la particule est constituée de 0,1 à 3 % en poids d'or, de 25 à 99,8 % en poids de $TiO_2$, de 0 à 50 % en poids d'oxydes de silicium, de 0 à 25 % en poids d'$Al_2O_3$, de 0 à 25 % en poids d'oxydes de métaux alcalins, de métaux alcalino-terreux, de métaux des terres rares et/ou de zirconium, de 0 à 20 % en poids d'oxydes de zirconium, de fer, de zinc, de nickel et/ou de cobalt et de 0 à 5 % en poids d'autres composants,

**en ce que** l'or, éventuellement en même temps que des oxydes de fer, de zinc, de nickel et/ou de cobalt se présente sous forme de particules ayant un diamètre moyen entre 2 et 10 nm, liées à la particule de catalyseur, **en ce que** la particule de catalyseur présente un diamètre équivalent géométrique moyen entre 5 μm et 10 mm, et **en ce que** le procédé est mis en œuvre en continu, à l'aide d'une catalyse hétérogène en phase liquide.

**2.** Procédé selon la revendication 1, **caractérisé en ce que** la particule de catalyseur est constituée de 0,3 à 2,5 % en poids d'or, de 50 à 99,5 % en poids de $TiO_2$, de 0 à 20 % en poids d'oxydes de silicium, de 0 à 10 % en poids d'oxydes de fer, de zinc, de nickel et/ou de cobalt et de 0 à 5 % en poids d'autres composants.

**3.** Procédé selon la revendication 1 ou 2, **caractérisé en ce que** la particule de catalyseur est constituée de 0,3 à 2,5 % en poids d'or, de 96 à 99,5 % en poids de $TiO_2$ et au maximum de 3,7 % en poids d'autres composants, pour ce qui concerne lesquels il ne s'agit ni d'or, ni de $TiO_2$.

**4.** Procédé selon au moins l'une des revendications 1 à 3, **caractérisé en ce que** l'or et la particule contenant de l'or et des oxydes de fer, de zinc, de nickel et/ou de cobalt se présentent dans la zone extérieure de la particule de catalyseur avec un diamètre moyen entre 2 et 10 nm.

**5.** Procédé selon au moins l'une des revendications 1 à 3, **caractérisé en ce que** l'or et/ou les particules contenant de l'or et des oxydes de fer, de zinc, de nickel et/ou du cobalt se présentent, avec un diamètre moyen entre 2 et 10 nm, répartis uniformément sur la particule de catalyseur.

**6.** Procédé selon au moins l'une des revendications 1 à 5, **caractérisé en ce que** les particules de catalyseur sont poreuses, présentent une aire spécifique entre 20 et 300 $m^2/g$, et **en ce que** leur diamètre moyen de pore est de 2 à 30 nm.

**7.** Procédé selon la revendication 6, **caractérisé en ce que** l'aire spécifique de la particule de catalyseur est comprise entre 30 et 200 $m^2/g$, et **en ce que** le diamètre moyen des pores est de 5 à 25 nm.

**8.** Procédé selon au moins l'une des revendications 1 à 7, **caractérisé en ce que**, pour ce qui concerne le procédé, il s'agit d'une estérification oxydative d'un aldéhyde en présence d'oxygène et d'un alcool, conduisant à un ester d'acide carboxylique.

**9.** Procédé selon la revendication 8, **caractérisé en ce que**, pour ce qui concerne le procédé, il s'agit de l'estérification oxydative de méthacroléine en présence d'oxygène et d'un alcool pour donner un méthacrylate d'alkyle.

**10.** Procédé selon les revendications 1 et 9, **caractérisé en ce que** la réaction entre la méthacroléine et le méthanol est mise en œuvre en continu en présence d'un gaz oxygéné, dans le respect des paramètres suivants :

I. à une température de réaction entre 40 et 120 °C,
II. sous une pression absolue entre 1 et 20 bar,
III. présentant une densité en suspension du catalyseur, qui se présente agité et tourbillonné dans le mélange stationnaire de produits contenant du MMA, de la méthacroléine, du méthanol et de l'eau, entre 1 et 20 % en poids,

la matière première méthacroléine n'étant pas entièrement convertie dans au moins un réacteur, mais, après une conversion partielle entre 20 et 95 %, étant séparée en continu du catalyseur par filtration, et envoyée au traitement.

**11.** Procédé selon la revendication 10, **caractérisé en ce qu'**on envoie en continu au réacteur de l'air en tant que gaz oxygéné, la teneur en oxygène de la phase gazeuse qui se forme et qui se trouve au-dessus du mélange réactionnel étant entre 2 et 8 % en volume.

**12.** Procédé selon la revendication 10 ou 11, **caractérisé en ce que** la réaction est réalisée à une température de réaction entre 50 et 100 °C sous une pression absolue entre 2 et 15 bar.

**13.** Procédé selon au moins l'une des revendications 10 à 12, **caractérisé en ce que** la densité en suspension du catalyseur particulaire est comprise entre 2 et 15 % en poids.

**14.** Procédé selon au moins l'une des revendications 10 à 13, **caractérisé en ce que** la matière première méthacroléine n'est pas convertie en totalité dans un réacteur ou dans plusieurs réacteurs montés en série, mais, après une

conversion partielle entre 40 et 80 %, est séparée en continu du catalyseur par filtration et envoyée à un traitement.

Abb. 1

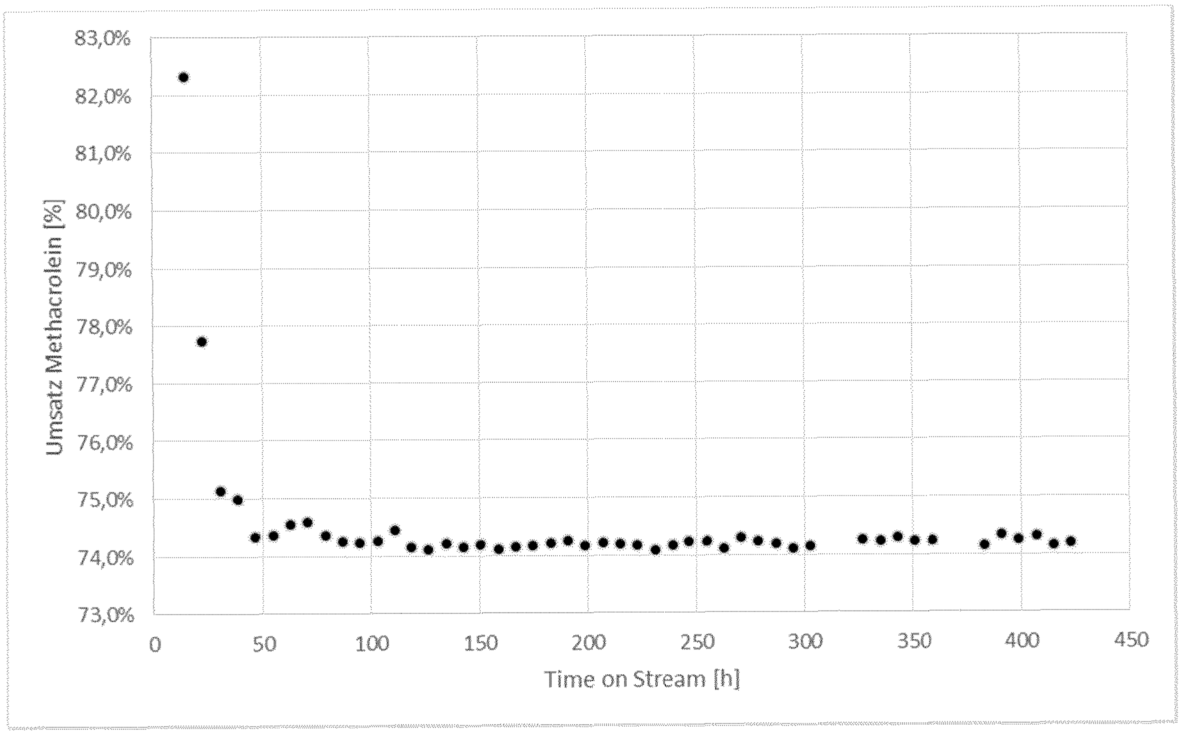
83,0%
82,0%
81,0%
80,0%
79,0%
78,0%
77,0%
76,0%
75,0%
74,0%
73,0%
Umsatz Methacrolein [%]
0
50
100
150
200
250
300
350
400
450
Time on Stream [h]

Abb. 2

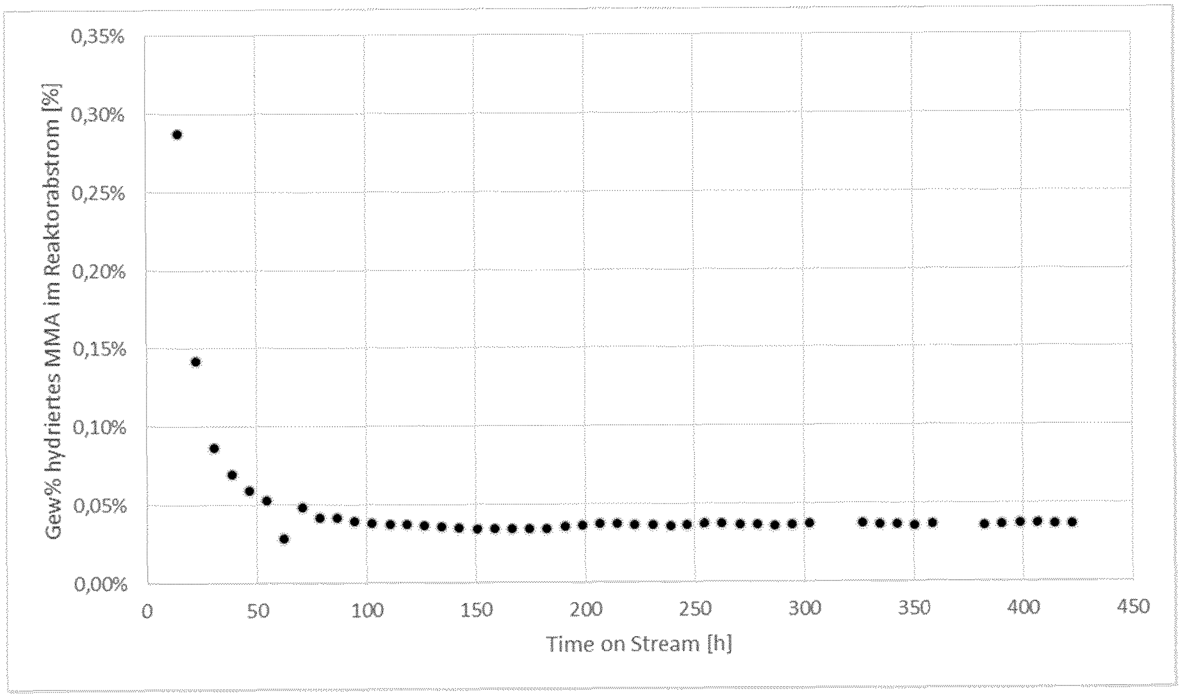
0,35%
0,30%
0,25%
0,20%
0,15%
0,10%
0,05%
0,00%
Gew% hydriertes MMA im Reaktorabstrom [%]
0
50
100
150
200
250
300
350
400
450
Time on Stream [h]

Abb. 3

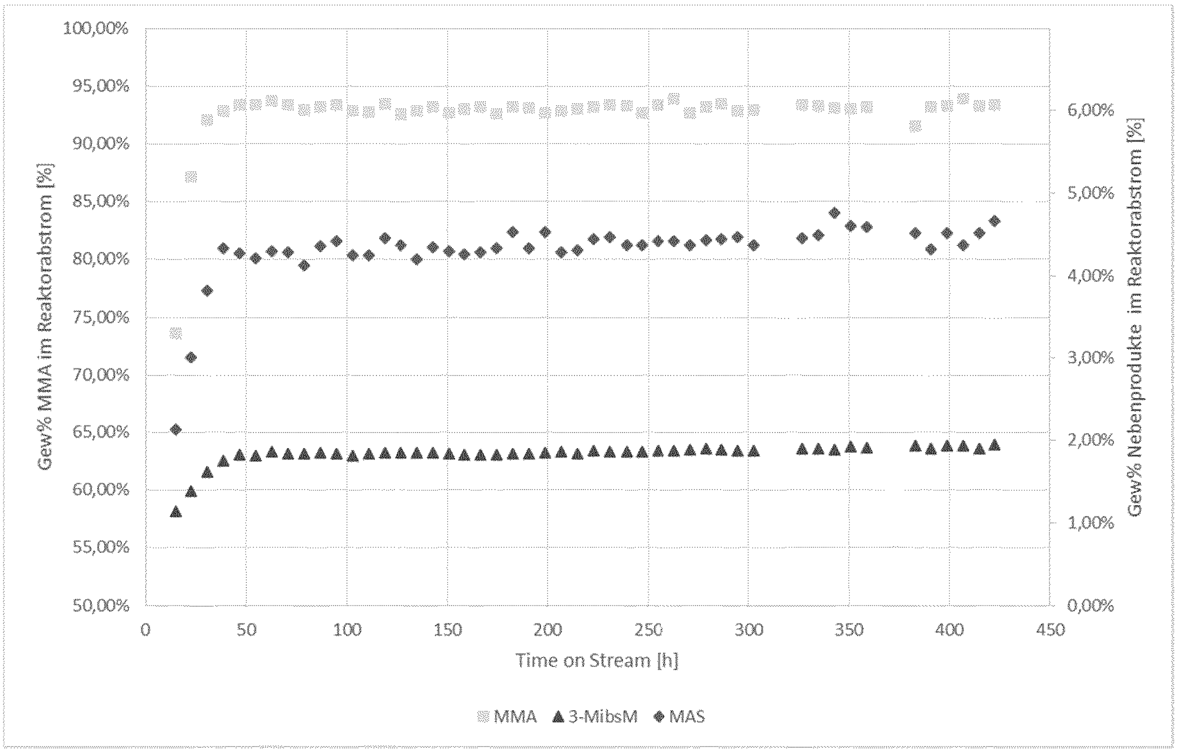
100,00%
95,00%
90,00%
85,00%
80,00%
75,00%
70,00%
65,00%
60,00%
55,00%
50,00%
Gew% MMA im Reaktorabstrom [%]
6,00%
5,00%
4,00%
3,00%
2,00%
1,00%
0,00%
Gew% Nebenprodukte im Reaktorabstrom [%]
0 50 100 150 200 250 300 350 400 450
Time on Stream [h]
MMA
3-MibsM
MAS

Abb. 4

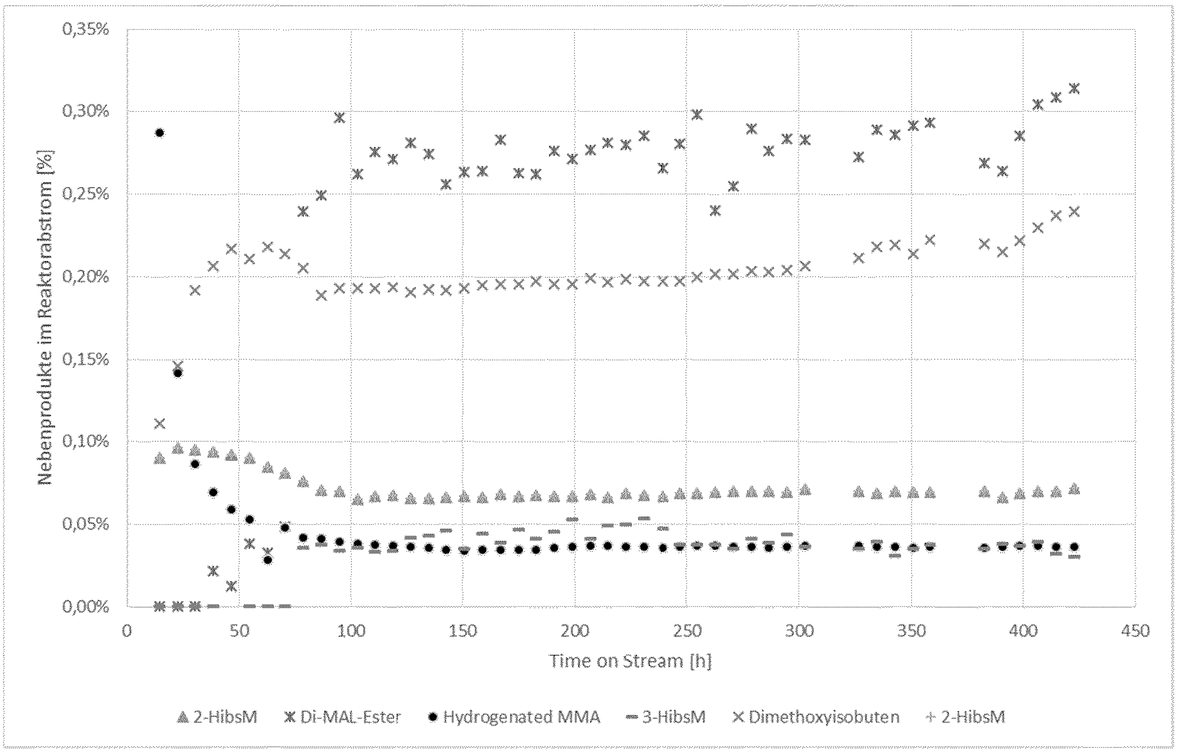
0,35%
0,30%
0,25%
0,20%
0,15%
0,10%
0,05%
0,00%
Nebenprodukte im Reaktorabstrom [%]
0 50 100 150 200 250 300 350 400 450
Time on Stream [h]
2-HibsM
Di-MAL-Ester
Hydrogenated MMA
3-HibsM
Dimethoxyisobuten
2-HibsM

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5969178 A
- US 7012039 B
- EP 1393800 A
- US 6040472 A
- EP 2177267 A
- EP 2210664 A
- WO 2017084969 A
- EP 0092097 A
- DE 2855504
- EP 2210664 A1

### In der Beschreibung aufgeführte Nicht-Patentliteratur

- *CHEMCATCHEM,* 2017, vol. 9 (11), 1960-1968
- *Microporous and mesoporous materials,* vol. 237 (1016), 12-22
- **HARUTA et al.** *J. Catal.,* 1993, vol. 144, 175-192
- **XIAOYUE WAN.** *Catalysis today,* 2014, vol. 233, 147
- **C. MARSDEN ; C. H. CHRISTENSEN et al.** *Green Chem.,* 2008, vol. 10, 168-170
- **KEN SUZUKI.** *ACS Catalysis,* 2013, vol. 3 (8), 1845
- **RANEY NICKEL ; RANEY COBALT.** Lehrbuch der Anorganischen Chemie. Gruyter
- Methacrylic Acid from Ethylene, and Trends and Future of Monomer-MMA Technologies. Ullmann’s Encyclopedia of Industrial Chemistry 2012. SUMITOMO KAGAKU, 2004